# EUROPEAN PATENT SPECIFICATION

(11) **EP 4 271 431 B1**
(45) Date of publication and mention of the grant of the patent: **09.04.2025**
(21) Application number: 22705315.4
(22) Date of filing: 03.02.2022
(51) Int. Cl.: A61L 29/04, A61L 29/14, A61L 29/18

(54) **INFLATABLE MEDICAL BALLOONS**
AUFBLASBARE MEDIZINISCHE BALLONS
BALLONNETS MÉDICAUX GONFLABLES

(30) Priority: 09.02.2021 US 202163147687 P
(43) Date of publication of application: 08.11.2023
(73) Proprietor: Becton, Dickinson and Company, Franklin Lakes, NJ 07417-1800 (US)
(72) Inventor: LEE, Jeong, Soo, Irvine, CA 92614 (US)
(74) Representative: Venner, Julia Ann
(86) International application number: PCT/US2022/015133
(87) International publication number: WO 2022/173654

(56) References cited:
- US-A1- 2014 142 505
- US-A1- 2015 290 435
- US-A1- 2016 243 342

## Description

### BACKGROUND

### Related Applications

This application claims priority based on United States Provisional Patent Application Serial No. 63/147,687, filed February 9, 2021 and entitled INFLATABLE MEDICAL BALLOONS.

### Field

The present disclosure generally relates to the field of inflatable balloons used in the minimally invasive transcatheter medical procedures.

### Description of Related Art

An inflatable balloon associated with a catheter can be positioned into various body lumens, vessels, and/or chambers in any number of medical procedures. Inflatable balloons can be used in minimally invasive transcatheter procedures. Inflatable balloons can be used in procedures for diagnosing any number of conditions, aid surgical procedures and/or selection of appropriate medical treatments.

US 2015/0290435 describes a balloon catheter comprises an elongate catheter shaft having a proximal section, a distal section, and an inflation lumen, and a multilayer balloon on the distal section of the shaft. The multilayer balloon comprises a first layer made of a first polymer material having a first Shore durometer hardness, a second layer made of a second polymer material having a second Shore durometer hardness lower than the first shore durometer hardness, wherein the second layer is an inner layer relative to the first layer, and an outer-most layer made of a third polymer material having a third Shore durometer hardness lower than the second Shore durometer hardness. US 2014/0142505 and US 2016/0243342 also describe multilayer balloons for catheters.

### SUMMARY

Aspects of the presently claimed invention are set out in the independent claims. Particular embodiments of these aspects are set out in the dependent claims. Any subject matter contained herein that does not fall within the scope of the appended claims is considered as being useful for understanding the invention.

Described herein are devices, systems, and methods relating to medical delivery systems comprising a balloon catheter and an inflatable balloon associated therewith, and an introducer catheter configured to slidably receive at least a portion of the balloon catheter. The inflatable balloon can comprise a balloon wall configured to facilitate retraction of the inflatable balloon in a deflated state back into the introducer catheter.

In some implementations useful for understanding the presently claimed invention, a medical delivery system can comprise a balloon catheter, and an inflatable balloon associated with a portion of the balloon catheter. The inflatable balloon can comprise a balloon wall at least partially defining an inflated volume of the inflatable balloon, the balloon wall comprising a first balloon wall layer and a second balloon wall layer, the first balloon wall layer comprising a Shore A durometer hardness value smaller than that of the second balloon wall layer. The first balloon wall layer can be an inner layer of the balloon wall relative to the second balloon wall layer.

In some embodiments, the first balloon wall layer comprises a Shore A durometer hardness value of about 20 to about 45 and the second balloon wall layer comprises a Shore A durometer hardness value of about 25 to about 50.

In some embodiments, the balloon wall is the first balloon wall layer and the second balloon wall layer. In some embodiments, the first balloon wall layer is about 70% to about 85% of a total thickness of the balloon wall.

In some embodiments, at least one of the first balloon wall layer and the second balloon wall layer comprises a styrenic block copolymer. In some embodiments, the styrenic block copolymer is styrene-isoprene-styrene (SIS). In some embodiments, the styrenic block copolymer is one of styrene-butadiene-styrene (SBS) or styrene-ethylene-butylene-styrene (SEBS). In some embodiments, the styrenic block copolymer comprises about 5 mol % to about 15 mol % of styrene monomers.

In some embodiments, the balloon wall comprises a surface tackiness reducing agent. In some embodiments, the surface tackiness reducing agent comprises polytetrafluoroethylene (PTFE). In some embodiments, the balloon wall comprises about 5 wt% to about 10 wt % of the polytetrafluoroethylene (PTFE). In some embodiments, the surface tackiness reducing agent comprises a radiopaque filler material. In some embodiments, the balloon wall comprises about 10 wt% to about 40 wt % of the radiopaque filler material. In some embodiments, the radiopaque filler material comprises at least one of bismuth oxychloride (BiOCl), barium sulfate (BaSO₄), and bismuth subcarbonate ((BiO)₂CO₃). In some embodiments, the second balloon wall layer comprises the surface tackiness reducing agent.

In some embodiments, at least one of the first balloon wall layer and the second balloon wall layer comprises polyurethane.

In some embodiments, the inflatable balloon is around a distal portion of the balloon catheter. In some embodiments, the balloon wall comprises a proximal portion and a distal portion, both the proximal portion and the distal portion being bonded circumferentially around the distal portion of the balloon catheter to provide a circumferential balloon around the distal portion. In some embodiments, the balloon wall is bonded circumferentially around the distal portion of the balloon catheter, the inflated volume being defined by a circumferential wall portion of the distal portion to which the balloon wall is bonded and the balloon wall.

In some embodiments, the balloon wall comprises a third balloon wall layer, the third balloon wall layer being an inner balloon wall layer, the second balloon wall layer being an outer balloon wall layer, and the first balloon wall layer being an intermediate balloon wall layer between the third balloon wall layer and the second balloon wall layer. In some embodiments, the first balloon wall layer and the second balloon wall layer each comprise a respective styrenic block copolymer, and wherein the third balloon wall layer comprises polyurethane.

In some embodiments, the system can further comprise an introducer catheter, at least the portion of the balloon catheter being configured to be slidably received within a balloon catheter lumen of the introducer catheter. In some embodiments, the introducer catheter has an outer diameter less than about 0.5 French larger than that of the balloon catheter. In some embodiments, the portion of the balloon catheter associated with the inflatable balloon and the inflatable balloon in a deflated state are configured to be extendable and retractable through an opening at a distal end of the introducer catheter.

In some implementations useful for understanding the presently claimed invention, a method of assembling a medical balloon catheter system can comprise providing a first inflatable balloon in an uninflated state, the first inflatable balloon comprising a first balloon wall; inflating the first inflatable balloon from the uninflated state to an inflated state to achieve a strain hardening limit of the first balloon wall, the inflated state comprising an inflated volume and the first balloon wall at least partially defining the inflated volume; and deflating the first inflatable balloon from the inflated state to a deflated state, a size of the first inflatable balloon in the deflated state being larger than that in the uninflated state. The method can include forming a second inflatable balloon associated with a balloon catheter using a first balloon wall portion.

In some embodiments, forming the second inflatable balloon comprises positioning the first balloon wall portion around a distal portion of the balloon catheter, and coupling a proximal circumferential portion and a distal circumferential portion of the first balloon wall portion to the distal portion of the balloon catheter, a medial circumferential portion of the first balloon wall portion being expandable upon inflation.

In some embodiments, providing the first inflatable balloon comprises providing a first elongate inflatable balloon, the first elongate inflatable balloon comprising a longitudinal dimension and a lateral dimension perpendicular to the longitudinal dimension. Forming the second inflatable balloon can comprise cutting the first elongate inflatable balloon along two paths parallel to the lateral dimension and spaced apart from one another to provide the first balloon wall portion, the first balloon wall portion comprising a circumferential portion of the first elongate inflatable balloon and having a longitudinal dimension shorter than that of the first elongate balloon. The first balloon wall portion can be positioned around a distal portion of the balloon catheter. A proximal circumferential portion and a distal circumferential portion of the first balloon wall portion can be bonded to the distal portion of the balloon catheter, a medial circumferential portion of the first balloon wall portion being expandable upon inflation. In some embodiments, providing the first elongate inflatable balloon comprises providing a cylindrical balloon, and wherein cutting the first elongate inflatable balloon comprises providing a cylindrical balloon wall portion having a longitudinal dimension shorter than that of the first elongate inflatable balloon.

In some embodiments, the method further comprises inserting the distal portion of the balloon catheter through a balloon catheter lumen of an introducer catheter, the introducer catheter comprising an outer diameter size less than about 0.5 French larger than that of the balloon catheter.

In some embodiments, providing the first inflatable balloon comprises providing a first elongate inflatable balloon, the first elongate inflatable balloon comprising a longitudinal dimension and a lateral dimension perpendicular to the longitudinal dimension, and wherein deflating the first inflatable balloon from the inflated state to the deflated state comprises deflating the first elongate inflatable balloon from the inflated state to a deflated state comprising a lateral dimension about 15% to about 20% larger than that of a lateral dimension of the first elongate inflatable balloon in the uninflated state.

In some embodiments, the method can further comprise providing a first composition comprising styrene and one of isoprene or butadiene, and extruding the first composition to provide the first balloon wall of the first inflatable balloon, the first balloon wall comprising a styrenic block copolymer. In some embodiments, providing the first composition comprises providing a composition comprising about 5 mol % to about 15 mol % of styrene monomers. In some embodiments, providing the first composition comprises providing a surface tackiness reducing agent, the surface tackiness reducing agent comprising at least one of polytetrafluoroethylene (PTFE) and a radiopaque filler material. In some embodiments, the radiopaque filler material comprises at least one of bismuth oxychloride (BiOCl), barium sulfate (BaSO₄), and bismuth subcarbonate ((BiO)₂CO₃).

In some embodiments, the method can further comprise providing a first composition comprising polyurethane, and extruding the first composition to provide the first balloon wall of the first inflatable balloon.

In some embodiments, the method can further comprise providing a first composition comprising styrene and one of isoprene or butadiene; providing a second composition comprising styrene and one of isoprene or butadiene, the second composition being different from the first composition; and co-extruding the first composition and the second composition to provide a first balloon wall comprising a first balloon wall layer and a second balloon wall layer, respectively, the first balloon wall layer being an inner layer of the first balloon wall and the second balloon wall layer being an outer layer of the first balloon wall, the first balloon wall layer comprising a Shore A hardness smaller than that of the second balloon wall layer.

### BRIEF DESCRIPTION OF THE DRAWINGS

Throughout the drawings, reference numbers may be reused to indicate correspondence between reference elements. However, it should be understood that the use of similar reference numbers in connection with multiple drawings does not necessarily imply similarity between respective embodiments associated therewith. Furthermore, it should be understood that the features of the respective drawings are not necessarily drawn to scale, and the illustrated sizes thereof are presented for the purpose of illustration of inventive aspects thereof. Generally, certain of the illustrated features may be relatively smaller than as illustrated in some embodiments or configurations.
Figure 1 is a cross-sectional view of a human heart.
Figures 2A and 2B show an example of a medical delivery system in accordance with one or more embodiments.
Figure 3A shows the medical delivery system of Figure 2 where the inflatable balloon is in an inflated state, and Figure 3B shows the inflatable balloon in a deflated state. Figure 3C shows retraction of the inflatable balloon in the deflated state back into an introducer catheter.
Figure 4 shows the medical delivery system of Figures 2 and 3 positioned into the heart.
Figure 5 is a flow diagram of an example of a process for manufacturing an example of a medical delivery system.
Figure 6 is a flow diagram of an example of a process for assembling an example of a medical delivery system.
Figure 7 shows an example of a medical delivery system assembled according to the process of Figure 6, in accordance with one or more embodiments.

### DETAILED DESCRIPTION

The headings provided herein are for convenience only and do not necessarily affect the scope or meaning of the claimed invention.

The present disclosure relates to devices, systems and methods for medical delivery systems comprising a balloon catheter and an inflatable balloon associated therewith, and an introducer catheter configured to slidably receive at least a portion of the balloon catheter. The portion of the balloon catheter associated with the inflatable balloon and the inflatable balloon in an uninflated and/or deflated state can be received within the introducer catheter. The portion of the balloon catheter associated with the inflatable balloon can be released from the introducer catheter at a target site and the inflatable balloon can be subsequently inflated. After the desired procedure is completed, the inflatable balloon can be deflated for retraction back into the introducer catheter. The inflatable balloon can comprise a balloon wall configured to facilitate retraction of the inflatable balloon in the deflated state back into the introducer catheter.

Certain standard anatomical terms of location are used herein to refer to the anatomy of animals, and namely humans, with respect to the preferred embodiments. Although certain spatially relative terms, such as "outer," "inner," "upper," "lower," "below," "above," "vertical," "horizontal," "top," "bottom," and similar terms, are used herein to describe a spatial relationship of one device/element or anatomical structure to another device/element or anatomical structure, it is understood that these terms are used herein for ease of description to describe the positional relationship between element(s)/structures(s), as illustrated in the drawings. It should be understood that spatially relative terms are intended to encompass different orientations of the element(s)/structures(s), in use or operation, in addition to the orientations depicted in the drawings. For example, an element/structure described as "above" another element/structure may represent a position that is below or beside such other element/structure with respect to alternate orientations of the subject patient or element/structure, and vice-versa.

Inflatable balloons can be used in various minimally invasive medical procedures. Inflatable balloons can be associated with a balloon catheter and the balloon catheter can be positioned within a body lumen, vessel, and/or chamber to locate the inflatable balloon at a desired site within the body. The balloon catheter can at least be partially received within an introducer catheter, such as to facilitate advancement of the balloon catheter along tortuous anatomical pathways. Generally, an inflatable balloon and at least the associated portion of the balloon catheter can be deployed from the introducer catheter. The inflatable balloon can then be inflated at a desired location within the body. The inflatable balloon can be subsequently deflated and retracted back into the introducer catheter, such as for removal from within the body. Typical inflatable balloons can comprise a balloon wall which can tend to stick against one or more surfaces of the introducer catheter. A typical balloon wall can demonstrate plastic deformation after initial inflation of the inflatable balloon. The plastic deformation of the balloon wall can lead to bagginess of the balloon wall after subsequent deflation. For example, one or more portions of a typical inflatable balloon can adhere to and/or get pinched by one or more portions of the introducer catheter, impeding desired retraction of the deflated balloon. In some cases, the inflatable balloon can thereby be damaged and/or the balloon catheter may not be properly retracted back into the introducer catheter.

Described herein are systems, devices and methods relating to medical delivery systems comprising a balloon catheter and an inflatable balloon associated therewith, and an introducer catheter configured to slidably receive at least a portion of the balloon catheter, including the portion of the balloon catheter associated with the inflatable balloon. The inflatable balloon can comprise a balloon wall which has a plurality of balloon wall layers. For example, the balloon wall can comprise two balloon wall layers. The balloon wall can be a two wall-layer balloon wall. In some embodiments, the balloon wall can have an inner balloon wall layer and an outer balloon wall layer. The outer balloon wall layer can have a Shore A durometer hardness value greater than that of the inner balloon wall layer. The outer balloon wall layer can comprise a styrenic block co-polymer and one or more tackiness reducing agents. For example, the outer balloon wall layer can comprise one of styrene-isoprene-styrene (SIS), styrene-butadiene-styrene (SBS) or styrene-ethylene-butylene-styrene (SEBS). In some embodiments, the inner balloon wall layer can comprise a styrenic block co-polymer. In some embodiments, the inner balloon wall layer can be a styrene-isoprene-styrene (SIS) layer, a styrene-butadiene-styrene (SBS) layer or a styrene-ethylene-butylene-styrene (SEBS) layer. The inflatable balloon can be delivered to a target site in an uninflated state, such as having never been previously inflated. The inflatable balloon can be released from the introducer catheter and inflated at the target site. After completion of the medical procedure, the inflatable balloon can be deflated for retraction back into the introducer catheter.

The harder outer balloon wall layer can demonstrate a reduced tackiness, thereby providing a balloon wall comprising an outer surface demonstrating a reduced tackiness to facilitate retraction of a deflated inflatable balloon back into the introducer catheter. A reduced tackiness can advantageously facilitate sliding of the deflated inflatable balloon against one or more surfaces of the introducer catheter such that the balloon wall does not become stuck to the introducer catheter. In some cases, including the embodiments of the presently claimed invention, the inner balloon wall layer can have a thickness greater than that of the outer balloon wall layer. A balloon wall comprising the outer balloon wall layer and inner balloon wall layer as described herein, and having the described relative thickness of the outer balloon wall layer and inner balloon wall layer, can facilitate desired retraction of the inflatable balloon in a deflated state, while maintaining other desired characteristics of the inflatable balloon while the inflatable balloon is in an inflated state.

An inflatable balloon can comprise a balloon wall which has undergone a pre-inflation process. A balloon wall which had undergone a pre-inflation process can demonstrate less plastic deformation when subsequently inflated, such as compared to a balloon wall which has never been previously inflated. Reduced plastic deformation of the balloon wall in subsequent inflations can result in less slack and/or bagginess when the inflatable balloon deflated after an inflation. Less slack and/or bagginess in the deflated state can facilitate retraction of the deflated balloon and the balloon catheter back into an introducer catheter.

Although the inflatable balloons are primarily described herein as being deployed into the heart, it will be understood that the use of the inflatable balloons are not so limited. The inflatable balloons can be positioned within any number of vessels, lumens, and/or chambers within the body.

The term "associated with" is used herein according to its broad and ordinary meaning. For example, where a first feature, element, component, device, or member is described as being "associated with" a second feature, element, component, device, or member, such description should be understood as indicating that the first feature, element, component, device, or member is physically coupled, attached, or connected to, integrated with, embedded at least partially within, or otherwise physically related to the second feature, element, component, device, or member, whether directly or indirectly.

Reference herein to "catheters" and/or "delivery catheters" can refer or apply generally to any type of elongate tubular delivery device comprising an inner lumen configured to slidably receive instrumentation, such as for positioning within an atrium or ventricle, including for example delivery sheaths and/or cannulas.

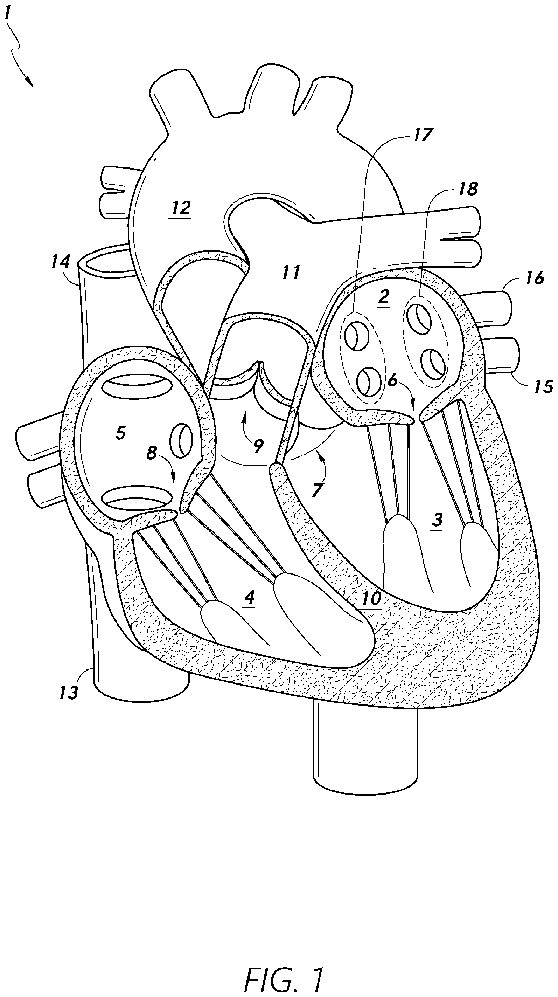

Figure 1 is a cross-sectional view of an example heart 1 having various features/anatomy relevant to certain aspects of the present inventive disclosure. The heart 1 includes four chambers, namely the left ventricle 3, the left atrium 2, the right ventricle 4, and the right atrium 5. A wall of muscle, referred to as the septum 10, separates the left-side chambers from the right-side chambers. An atrial septum wall portion separates the left atrium 2 from the right atrium 5, whereas a ventricular septum wall portion separates the left ventricle 3 from the right ventricle 4. Blood flow through the heart 1 is at least partially controlled by four valves, the mitral valve 6, aortic valve 7, tricuspid valve 8, and pulmonary valve 9. The mitral valve 6 separates the left atrium 2 and the left ventricle 3 and controls blood flow therebetween. The aortic valve 7 separates and controls blood flow between the left ventricle 3 and the aorta 12. The tricuspid valve 8 separates the right atrium 5 and the right ventricle 4 and controls blood flow therebetween. The pulmonary valve 9 separates the right ventricle 4 and the pulmonary artery 11, controlling blood flow therebetween. The mitral valve 6 separates the left atrium 2 from the left ventricle 3. The aortic valve 7 separates the left ventricle 3 from the aorta 12.

The four valves of the heart aid the circulation of blood in the heart. The tricuspid valve 8 generally closes during ventricular contraction (i.e., systole) and opens during ventricular expansion (i.e., diastole). The pulmonary valve 9 separates the right ventricle 4 from the pulmonary artery 11 and generally is configured to open during systole so that blood may be pumped toward the lungs from the right ventricle 4, and close during diastole to prevent blood from leaking back into the right ventricle 4 from the pulmonary artery. The mitral valve 6 may generally be configured to open during diastole so that blood in the left atrium 2 can flow into the left ventricle 3, and close during diastole to prevent blood from leaking back into the left atrium 2. The aortic valve 7 is configured to open during systole to allow blood leaving the left ventricle 3 to enter the aorta 12, and close during diastole to prevent blood from leaking back into the left ventricle 3.

Deoxygenated blood enters the right atrium 5 through the inferior 13 and superior 14 venae cavae. The right side of the heart then pumps this deoxygenated blood into the pulmonary arteries around the lungs. There, fresh oxygen enters the blood stream, and the blood moves to the left side of the heart via a network of pulmonary veins ultimately terminating at the left atrium 2, as shown. The primary roles of the left atrium 2 are to act as a holding chamber for blood returning from the lungs (not shown) and to act as a pump to transport blood to other areas of the heart. The left atrium 2 receives oxygenated blood from the lungs via the pulmonary veins 15, 16. The ostia 17, 18 of the pulmonary veins 15, 16 are generally located at or near posterior left atrial wall of the left atrium 2. The oxygenated blood that is collected from the pulmonary veins 15, 16 in the left atrium 2 enters the left ventricle 3 through the mitral valve 6.

Various parameters of blood flow to or from the heart can be measured to determine the health status of a patient. Continuous monitoring of the various parameters can be performed to assess real-time health status of patients. Continuous monitoring can be used in diagnosis of any number of conditions, aid surgical procedures and/or selection of appropriate medical treatments. Inflatable balloons and the associated balloon catheters can be deployed to one or more lumens, vessels and/or chambers in the heart and/or around the heart to facilitate the continuous monitoring.

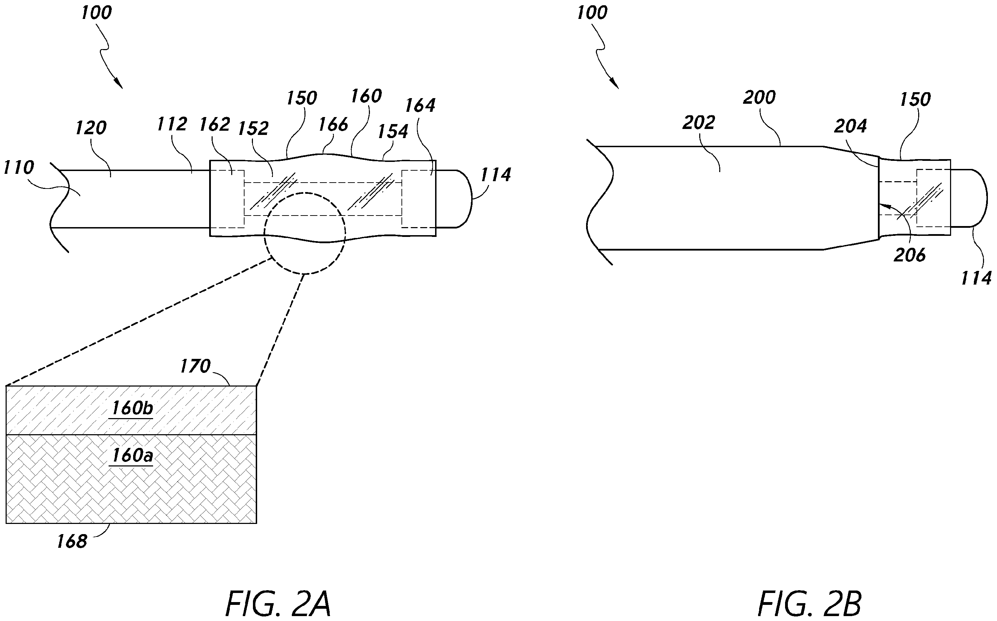

Figures 2A and 2B show an example of a medical delivery system 100 comprising an introducer catheter 200, and a balloon catheter 110 having an inflatable balloon 150 associated therewith. The inflatable balloon 150 can comprise a balloon wall 160. At least a portion of the balloon catheter 110 can be slidably received within a balloon catheter lumen of the introducer catheter 200. Figure 2A shows the balloon catheter 110 and Figure 2B shows a portion of the balloon catheter 110 received within the balloon catheter lumen of the introducer catheter 200. The inflatable balloon 150 is shown in Figures 2A and 2B in an uninflated state. The inflatable balloon 150 as shown can be in a state prior to an initial inflation, for example the inflatable balloon 150 never having been previously inflated. The balloon wall 160 of the inflatable balloon 150 never having been subjected to inflation. As described in further detail herein, the balloon wall 160 can demonstrate plastic deformation after an initial inflation of the inflatable balloon 150. Plastic deformation of the balloon wall 160 can result in a baggy balloon after the inflatable balloon 150 is deflated.

In some embodiments, the inflatable balloon 150 can be associated with a distal portion 112 of the balloon catheter 110. For example, the inflatable balloon 150 can be circumferentially around the distal portion 112 of the balloon catheter 110. The inflatable balloon 150 can comprise a balloon wall 160. In some embodiments, the balloon wall 160 can comprise one or more portions coupled to the balloon catheter 110 so as to form the inflatable balloon 150. The balloon wall 160 can at least partially define an inflated volume of the inflatable balloon 150. For example, the inflated volume can be defined by a portion of the balloon wall 160 and a portion of the balloon catheter 110. In some embodiments, the balloon wall 160 can comprise a proximal portion 162 and a distal portion 164 coupled to the balloon catheter 110. For example, the proximal portion 162 and the distal portion 164 can each be bonded circumferentially around respective parts of the balloon catheter distal portion 112 to provide a circumferential balloon around the distal portion 112. The balloon catheter 110 can have an exterior surface 120. The proximal portion 162 and the distal portion 164 can each be bonded to respective parts of the exterior surface 120 such that a medial portion 166 of the balloon wall 160 between the proximal and distal portions 162, 164 can partially define the inflated volume. In some embodiments, the inflated volume can be defined by the medial portion 166 and the portion of the balloon catheter exterior surface 120 over which the medial portion 166 is positioned.

Referring to Figure 2B, at least a portion of the balloon catheter 110 can be received within the introducer catheter 200. For example, at least the portion of the balloon catheter 110 with which the inflatable balloon 150 is associated can be slidably received within the balloon catheter lumen of the introducer catheter 200. Figure 2B shows the distal portion 112 of the balloon catheter 110 pre-loaded within the distal portion 202 of the introducer catheter. A portion of the inflatable balloon 150 can be distal of the distal end 204 of the introducer catheter 200. A portion of the balloon catheter 110 can be distal of the distal end 204 of the introducer catheter 200. For example, in a pre-loaded configuration, the distal end 114 of the balloon catheter 110 and a distal portion 154 of the inflatable balloon 150 can extending through a distal opening 206 at the distal end 204 of the introducer catheter 200. A proximal portion 152 of the inflatable balloon 150 can be received within the balloon catheter lumen of the introducer catheter 200. As described herein, the inflatable balloon 150 is shown in an uninflated state, for example having never been previously inflated.

The balloon wall 160 can comprise a plurality of layers. Referring back to Figure 2A, the balloon wall 160 can comprise a first balloon wall layer 160a and a second balloon wall layer 160b. For example, the balloon wall 160 can be the first balloon wall layer 160a and the second balloon wall layer 160b. The first balloon wall layer 160a can be adjacent to and in contact with the second balloon wall layer 160b. The first balloon wall layer 160a can be configured to be closer to the balloon catheter 110, for example an inner balloon wall layer. The second balloon wall layer 160b can be configured to be further from the balloon catheter 110, for example an outer balloon wall layer. The first balloon wall layer 160a can be adjacent to and in contact with the second balloon wall layer 160b such that the first balloon wall layer 160a can have a first surface 168 configured to be oriented toward the exterior surface 120 of the balloon catheter 110. The second balloon wall layer 160b can have a second surface 170 configured to be oriented away from the exterior surface 120 of the balloon catheter 110. For example, the first surface 168 can be an interior surface of the inflatable balloon 150 and the second surface 170 can be an exterior surface of the inflatable balloon 150. As described in further detail herein, the first balloon wall layer 160a can be made of a material different from that of the second balloon wall layer 160b. In some embodiments, an inflatable balloon having a balloon wall comprising a plurality of layers can facilitate retraction of the inflatable balloon in a deflated state back into an introducer catheter.

In some embodiments, the first and second balloon wall layers 160a, 160b can have different hardness values. For example, the first balloon wall layer 160a can be softer than the second balloon wall layer 160b. In the presently claimed invention, the first balloon wall layer 160a comprises a Shore A durometer hardness value less than that of the second balloon wall layer 160b. In the presently claimed invention, an inner layer is softer than an outer layer of the balloon wall 160. Shore A durometer hardness values can be determined according to ASTM D2240-15e1 Standard Test Method for Rubber Property-Durometer Hardness (available from ASTM International, West Conshohocken, PA, and accessible at https://www.astm.org/Standards/D2240).

In some embodiments, the first balloon wall layer 160a can have a Shore A durometer hardness value of about 10 to about 45. For example, the first balloon wall layer 160a can have a Shore A durometer hardness value of about 20 to about 45. In some embodiments, the first balloon wall layer 160a can have a Shore A durometer hardness value of about 25. In some embodiments, the second balloon wall layer 160b can have a Shore A durometer hardness value of about 25 to about 50. For example, the second balloon wall layer 160b can have a Shore A durometer hardness value of about 25 to about 45. In some embodiments, the second balloon wall layer 160b can have a Shore A durometer hardness value of about 35 to about 40. In some embodiments, the second balloon wall layer 160b can have a Shore A durometer hardness value of about 40.

In the presently claimed invention, the first balloon wall layer 160a is about 70% to about 85% of a total thickness of the balloon wall 160. In some embodiments, the first balloon wall layer 160a can be about 70% of a total thickness of the balloon wall 160. In some embodiments, the first balloon wall layer 160a can be about 85% of a total thickness of the balloon wall 160. The second balloon wall layer 160b can be the remaining thickness of the balloon wall 160. For example, the second balloon wall layer 160b can be about 10% to about 45% of a total thickness of the balloon wall 160, including about 10% to about 40%. In some embodiments, the second balloon wall layer 160b can be about 15% to about 30% of the total thickness.

In some embodiments, the balloon wall 160 can have a total thickness of about 0.005 inches (in) to about 0.015 inches (in). 1 inch corresponds to 2.54 cm. In some embodiments, the balloon wall 160 can have a total thickness of about 0.010 inches (in) to about 0.015 inches (in). In some embodiments, the balloon wall 160 can have a total thickness of about 0.012 inches (in). In some embodiments, the balloon wall 160 can have a total thickness of about 0.020 inches (in) to about 0.030 inches (in). In some embodiments, the inflatable balloon 150 can have an inner diameter prior to an initial inflation of about 0.045 inches (in) to about 0.085 inches (in), including about 0.060 inches (in) to about 0.070 inches (in), and about 0.065 inches (in). In some embodiments, the inflatable balloon 150 can have an inner diameter prior to an initial inflation of about 0.2 inches (in) to about 0.3 inches (in).

A balloon wall can demonstrate plastic deformation after initial inflation of the inflatable balloon. The plastic deformation of the balloon wall can lead to bagginess and/or sagginess of the balloon wall after subsequent deflation. In a typical deflated inflatable balloon, bagginess and/or slack in a balloon wall can contribute to the deflated inflatable balloon being caught by and/or stuck on a distal end of an introducer catheter as the deflated inflatable balloon is retracted back into the introducer catheter. In some embodiments, a balloon wall layer comprising an increased hardness can have a reduced tackiness. An inflatable balloon comprising an outer balloon wall layer having an increased hardness can provide an outer surface with reduced tackiness. Reduced tackiness can facilitate sliding of a baggy and/or stretched inflatable balloon in a deflated state back into the introducer catheter. The deflated inflatable balloon can exhibit reduced friction against one or more surfaces of the introducer catheter as it is translated proximally relative to the introducer catheter. The deflated inflatable balloon can advantageously slide against one or more surfaces of the introducer catheter such that the balloon wall does not become stuck to the introducer catheter, and can be successfully retracted back into the introducer catheter.

A balloon wall comprising a plurality of layers can have an outer surface demonstrating a reduced tackiness to facilitate retraction of a deflated inflatable balloon back into an introducer catheter, while maintaining other desired characteristics. For example, characteristics of the outer balloon wall layer can be selected to provide an outer surface demonstrating reduced tackiness, while characteristics of an inner balloon wall layer can be selected so as to provide a balloon wall which can demonstrate other desirable characteristics, including elasticity and/or strength. In some embodiments, compositions of each of the inner and outer balloon wall layers, and the relative thickness of the inner and outer balloon wall layers, can be selected based at least in part on a desired tackiness of the outer surface layer of the balloon wall, while providing a balloon wall which can demonstrate desired elasticity and/or strength.

In some embodiments, an inflatable balloon associated with a balloon catheter as described herein can be configured to be retractable back into an introducer catheter having an outer diameter less than about 0.5 French larger than that of the balloon catheter. In some embodiments, the balloon catheter and the introducer catheter can have the same or similar outer diameter. For example, the balloon catheter and the introducer catheter can have the same French size. In some embodiments, the inflatable balloon in a deflated state can be retracted back into the introducer catheter having the same French size as the balloon catheter with which the inflatable balloon is associated, such as without being damaged and/or getting stuck to the introducer catheter.

In some embodiments, one or both of the first and second balloon wall layers 160a, 160b can comprise a styrenic block copolymer (e.g., commercially available from Foster Corporation, Putnam, CT, USA). In some embodiments, the first balloon wall layer 160a can comprise a styrenic block copolymer different from that of the second balloon wall layer 160b. In some embodiments, the first and second balloon wall layers 160a, 160b can comprise the same styrenic block copolymer. In some embodiments, the styrenic block copolymer is styrene-isoprene-styrene (SIS). For example, the first and/or the second balloon wall layers 160a, 160b can comprise styrene-isoprene-styrene (SIS). In some embodiments, the styrenic block copolymer is one of styrene-butadiene-styrene (SBS) or styrene-ethylene-butylene-styrene (SEBS). For example, the first and/or the second balloon wall layers 160a, 160b can comprise one of styrene-butadiene-styrene (SBS) or styrene-ethylene-butylene-styrene (SEBS).

In some embodiments, at least one of the first balloon wall layer 160a and the second balloon wall layer 160b can comprise polyurethane (e.g., commercially available from Avient Corporation, Avon Lake, OH, USA). In some embodiments, the first balloon wall layer 160a can comprise a styrenic block copolymer as described herein and the second balloon wall layer 160b can comprise polyurethane. For example, the first balloon wall layer 160a can be one of a styrene-isoprene-styrene (SIS) layer, a styrene-butadiene-styrene (SBS) layer or a styrene-ethylene-butylene-styrene (SEBS) layer, and the second balloon wall layer 160b can comprise polyurethane. In some embodiments, the first balloon wall layer 160a can comprise polyurethane and the second balloon wall layer 160b can comprise a styrenic block copolymer (e.g., styrene-isoprene-styrene (SIS) layer, styrene-butadiene-styrene (SBS) or styrene-ethylene-butylene-styrene (SEBS)).

A styrenic content of the first and/or second balloon wall layers 160a, 160b can be selected based on desired characteristics of the balloon wall 160, including reduced tackiness of the outer surface of the inflatable balloon 150 and/or desired elasticity and/or strength of the balloon wall 160. In some embodiments, the styrenic block copolymer of the first and/or second balloon wall layers 160a, 160b can comprise no more than about 25 mol% of styrene monomers, for example about 5 mol% to about 25 mol %. In some embodiments, the styrenic block copolymer of the first and/or second balloon wall layers 160a, 160b can comprise about 5 mol % to about 15 mol % of styrene monomers. For example, a styrenic content of the second balloon wall layer 160b can be selected to provide the desired tackiness of the second surface 170. In some embodiments, the second balloon wall layer 160b can comprise about 5 mol% to about 25 mol %, including about 5 mol % to about 15 mol %, of the styrene monomers. An increased styrenic content can provide a balloon wall layer demonstrating an increased Shore A durometer hardness value. In some embodiments, the first balloon wall layer 160a can comprise less styrene content than the second balloon wall layer 160b.

In some embodiments, the balloon wall 160 can comprise a surface tackiness reducing agent. In some embodiments, the second balloon wall layer 160b can comprise a surface tackiness reducing agent. The surface tackiness reducing agent can be selected to further fine tune the tackiness of the outer surface of the inflatable balloon 150, such as the second surface 170 of the balloon wall 160. In some embodiments, the first balloon wall layer 160a may not have any surface tackiness reducing agents. In some embodiments, the surface tackiness reducing agent comprises polytetrafluoroethylene (PTFE). In some embodiments, the second balloon wall layer 160b comprises no more than about 10 wt % of the polytetrafluoroethylene (PTFE). In some embodiments, the second balloon wall layer 160b comprises about 1 wt% to about 10 wt % of the polytetrafluoroethylene (PTFE), including about 5 wt% to about 10 wt %. In some embodiments, the second balloon wall layer 160b comprises no more than about 5 vol% of the polytetrafluoroethylene (PTFE). For example, the second balloon wall layer 160b can comprise about 1 vol% to about 5 vol% of the polytetrafluoroethylene (PTFE), including about 2 vol% to about 5 vol%.

In some embodiments, the surface tackiness reducing agent can comprise a biocompatible inorganic material. In some embodiments, the surface tackiness reducing agent comprises a radiopaque material. The radiopaque material can comprise one or more of bismuth oxychloride (BiOCl), barium sulfate (BaSO₄), and bismuth subcarbonate ((BiO)₂CO₃). In some embodiments, the second balloon wall layer 160b comprises about 5 wt% to about 40 wt % of the biocompatible inorganic material. For example, the second balloon wall layer 160b can comprise one or more of bismuth oxychloride (BiOCl), barium sulfate (BaSO₄), and bismuth subcarbonate ((BiO)₂CO₃), the one or more of the bismuth oxychloride (BiOCl), barium sulfate (BaSO₄), and bismuth subcarbonate ((BiO)₂CO₃) being about 5 wt% to about 40 wt % in total. In some embodiments, the second balloon wall layer 160b comprises about 10 wt% to about 40 wt % of the biocompatible inorganic material, including about 20 wt% to about 40 wt %. In some embodiments, the second balloon wall layer 160b comprises about 1 vol % to about 10 vol % of the biocompatible inorganic material, including about 5 vol % to about 10 vol %.

In some embodiments, the first balloon wall layer 160a can be a styrenic block copolymer layer and the second balloon wall layer 160b can comprise a styrenic block copolymer and one or more tackiness reducing agents. In some embodiments, the first balloon wall layer 160a can be a styrene-isoprene-styrene (SIS) layer. In some embodiments, the second balloon wall layer 160b can comprise styrene-isoprene-styrene (SIS) and one or more tackiness reducing agents. For example, the first balloon wall layer 160a be a styrene-isoprene-styrene (SIS) layer and the second balloon wall layer 160b can comprise styrene-isoprene-styrene (SIS) and one or more tackiness reducing agents. In some embodiments, the first balloon wall layer 160a can be either a styrene-butadiene-styrene (SBS) layer or a styrene-ethylene-butylene-styrene (SEBS) layer. In some embodiments, the second balloon wall layer 160b can comprise either styrene-butadiene-styrene (SBS) or styrene-ethylene-butylene-styrene (SEBS), and one or more tackiness reducing agents. In some embodiments, the balloon wall 160 can comprise a first balloon wall layer 160a being a styrene-butadiene-styrene (SBS) layer and a second balloon wall layer 160b comprising styrene-butadiene-styrene (SBS) and one or more tackiness reducing agents. In some embodiments, the balloon wall 160 can comprise a first balloon wall layer 160a being a styrene-ethylene-butylene-styrene (SEBS) layer and a second balloon wall layer 160b comprising styrene-ethylene-butylene-styrene (SEBS) and one or more tackiness reducing agents. In some embodiments, the styrenic co-polymer of the first balloon wall layer 160a and the second balloon wall layer 160b can be different.

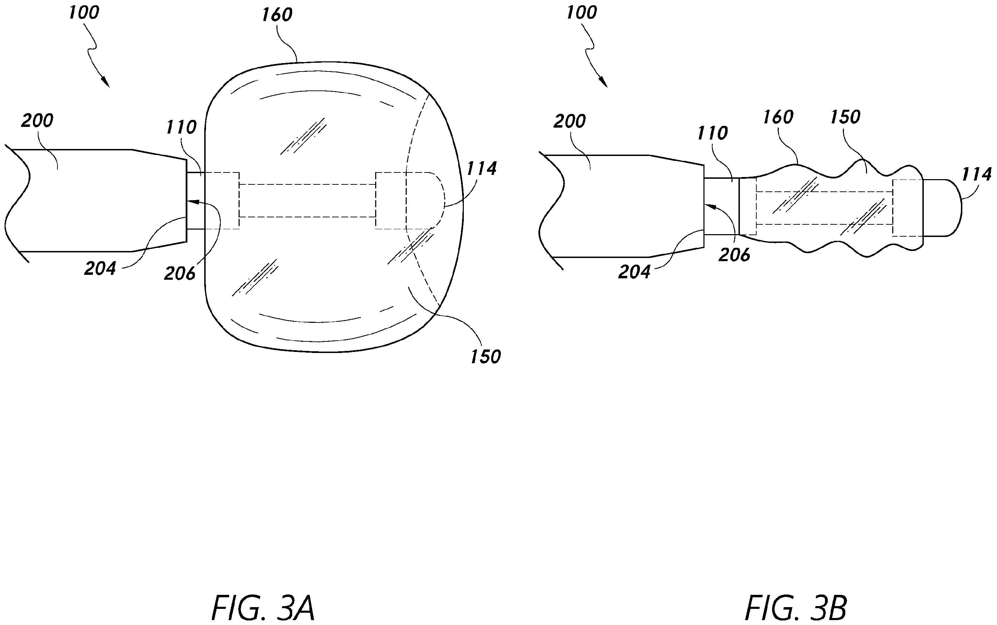

Figure 3A shows the medical delivery system 100 comprising the inflatable balloon 150 described with reference to Figures 2A and 2B in an inflated state. The inflatable balloon 150 can be carried by the introducer catheter 200 in an uninflated state to a desired position. After the introducer catheter 200 is positioned at a desired position, the portion of the balloon catheter 110 with which the inflatable balloon 150 is associated can be advanced distally and positioned distal of the distal end 204 of the introducer catheter 200. The For example, the portion of the balloon catheter 110 comprising the inflatable balloon 150 thereon can be extended distally through the distal opening 206 at the distal end 204 of the introducer catheter 200. Pressurized air can be injected into the inflatable balloon 150 for inflation. Subsequently, the inflatable balloon 150 can be deflated for retraction back into the introducer catheter 200.

Figure 3B shows the inflatable balloon 150 in a deflated state. As described herein, the inflatable balloon 150 can have never been previously inflated. Inflation at the target site can be an initial inflation of the inflatable balloon 150. The initial inflation and subsequent deflation can result in plastic deformation of the balloon wall 160. The balloon wall 160 can thereby exhibit a degree of irreversible stretch, resulting in the balloon wall 160 in the deflated state being a size larger than that prior to the initial inflation. As shown in Figure 3B, the inflatable balloon 150 can have some bagginess and/or slack in the deflated state.

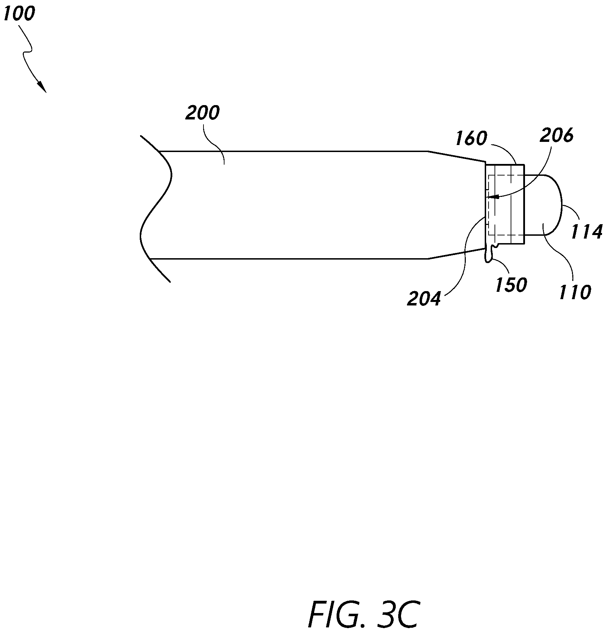

Figure 3C shows the balloon catheter 110 comprising the deflated inflatable balloon 150 being retracted back through the distal opening 206 into the introducer catheter 200. The inflatable balloon 150 can be retracted back into the introducer catheter 200 for removal from the patient. As shown in Figure 3C, due to the slack and/or bagginess of the inflatable balloon 150, a saggy portion of the balloon wall 160 can be between the distal end 204 of the introducer catheter 200 and the balloon catheter 110. The balloon wall 160 as described herein can facilitate withdrawal of the balloon catheter 110 without the inflatable balloon 150 being stuck to and/or caught on the distal end 204 of an introducer catheter 200.

In some embodiments, a balloon wall can comprise more than two balloon wall layers. In some embodiments, a balloon wall can comprise three balloon wall layers. For example, a balloon wall can comprise a first balloon wall layer, a second balloon wall layer and a third balloon wall layer. The first balloon wall layer can be an inner balloon wall layer. The second balloon wall layer can be a middle balloon wall layer. The third balloon wall layer can be an outer balloon wall layer. Material for the each of the layers can be selected to provide a balloon wall comprising an outer surface which can demonstrate a reduced tackiness, while also demonstrating desired elasticity, strength and/or manufacturability. In some embodiments, the first balloon wall layer can comprise a material amenable to bonding to a surface of the balloon catheter. In some embodiments, the third balloon wall layer can comprise a material comprising reduced tackiness. For example, the first balloon wall layer can comprise polyurethane. In some embodiments, the second balloon wall layer can be a styrenic block copolymer layer. In some embodiments, the third balloon wall layer can comprise a styrenic block copolymer and one or more tackiness reducing agents.

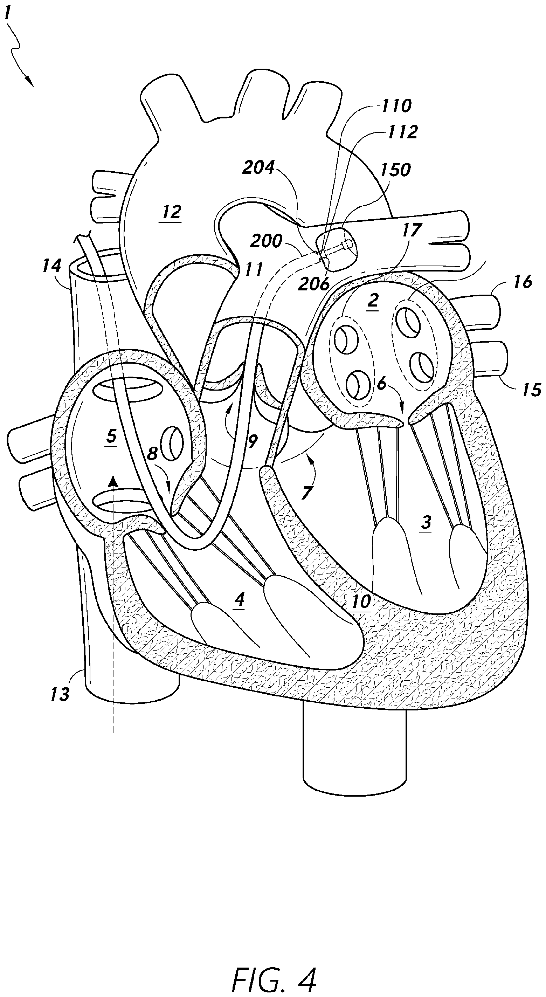

An inflatable balloon as described herein can be used in any number of different medical procedures. In some embodiments, an inflatable balloon can be used in a pulmonary artery catheterization process. For example, the balloon catheter 110 can be a Swan-Ganz catheter. Referring to Figure 4, the medical delivery system 100 is shown as being deployed at a target site within the heart 1. The introducer catheter 200 can be advanced through the inferior vena cava 13 or the superior vena cava 14 and into the right atrium 5. The introducer catheter 200 can be inserted through the tricuspid valve 8 from the right atrium 5 into the right ventricle 4. The introducer catheter 200 can be advanced through the pulmonary valve 9 from the right ventricle 4 such that the distal portion 202 of the introducer catheter 200 can be positioned into a pulmonary artery 11. After the distal portion 202 of the introducer catheter 200 is at a desired location within the pulmonary artery 11, the balloon catheter 110 can be translated distally relative to the introducer catheter 200 to position the inflatable balloon 150 distally of the distal end 204 of the introducer catheter 200. For example, the inflatable balloon 150 can be released from the distal opening 206 of the introducer catheter 200. The inflatable balloon 150 can subsequently be inflated, such as while positioned in the pulmonary artery 11. After inflation, the inflatable balloon 150 can follow the natural flow of the blood within the pulmonary artery 11. For example, the inflatable balloon 150 can move further downstream into the pulmonary artery 11 with the flow of the blood. The inflatable balloon 150 can stop once it is wedged within a pulmonary blood vessel. The distal portion 112 of the balloon catheter 110 can thereby be carried by the natural flow of blood into the pulmonary blood vessel. While the inflatable balloon 150 is wedged within the pulmonary blood vessel, measurements of a variety of parameters relating to cardiac function can be performed. In some embodiments, measurements of blood flow, blood pressure, and/or oxygen level can be collected. Continuous monitoring can be performed for blood flow, blood pressure and/or oxygen level, facilitating real-time decision making when treating patients.

The balloon catheter 110 can be pre-loaded in the introducer catheter 200 such that the balloon catheter 110 is delivered along with the introducer catheter 200 to the target site. In some embodiments, the introducer catheter 200 can be positioned at the target site and the balloon catheter 110 can be subsequently advanced along the balloon catheter lumen of the introducer catheter 200 to the target site.

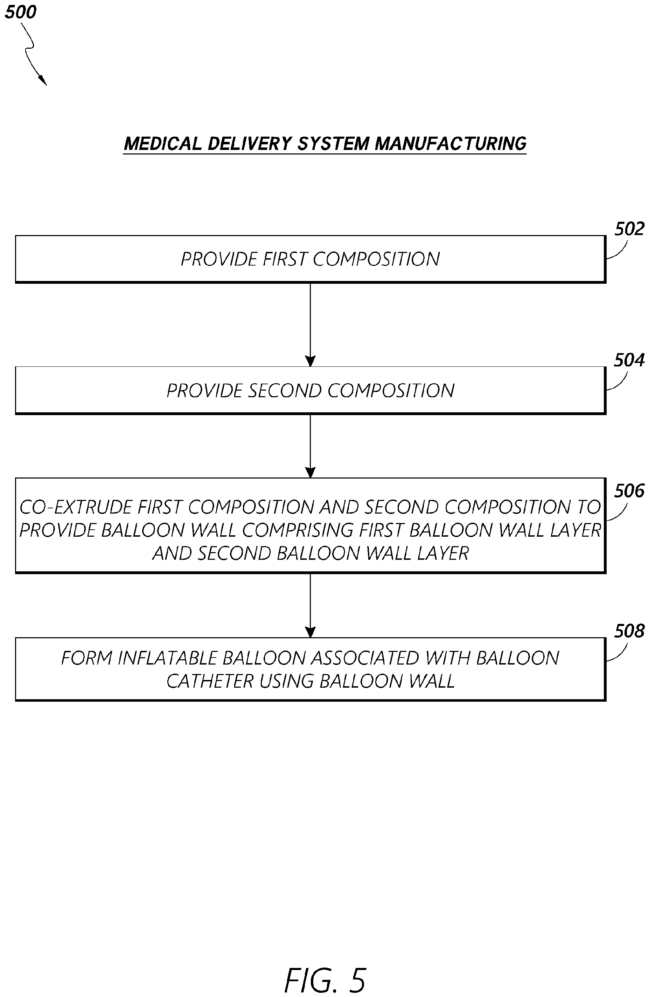

Figure 5 is a process flow diagram of an example of a process 500 for manufacturing a medical delivery system. The medical delivery system can comprise one or more features of the medical delivery system 100 as described with reference to Figures 2 through 4. In block 502, the process 500 can involve providing a first composition. In block 504, the process 500 can involve providing a second composition. The second composition can be different from the first composition. In block 506, the process 500 can involve co-extruding the first composition and the second composition to provide a balloon wall comprising a first balloon wall layer and a second balloon, respectively. The first and second compositions can be simultaneously extruded to provide the balloon wall made of the first and second layers. In block 508, the process 500 can involve forming an inflatable balloon associated with a balloon catheter using the balloon wall. The inflatable balloon can be formed on the balloon catheter, for example, to surround a portion of the balloon catheter.

In some embodiments, the co-extrusion process can comprise melting pellets of the first and second compositions. In some embodiments, the melted compositions can be passed through a die to form the first and second balloon wall layers. For example, the first composition can comprise one of styrene-isoprene-styrene (SIS), styrene-butadiene-styrene (SBS) or styrene-ethylene-butylene-styrene (SEBS). In some embodiments, the second composition can comprise one of styrene-isoprene-styrene (SIS), styrene-butadiene-styrene (SBS) or styrene-ethylene-butylene-styrene (SEBS), and one or more tackiness reducing agents as described herein. In some embodiments, the co-extrusion process can comprise a polymerization process. In some embodiments, the first and second compositions can each comprise precursors for forming one of styrene-isoprene-styrene (SIS), styrene-butadiene-styrene (SBS) or styrene-ethylene-butylene-styrene (SEBS). In some embodiments, the first and the second compositions can each comprise styrene and one of isoprene or butadiene. In some embodiments, the second composition can comprise one or more tackiness reducing agents, styrene, and one of isoprene or butadiene. The first and second compositions can be selected so as to provide a first balloon wall layer having a Shore A durometer hardness value smaller than that of the second balloon wall layer.

The balloon wall can comprise a tubular shape, including a cylindrical shape. For example, the first and second compositions can be extruded to form an elongate tube made of the first and second balloon wall layers. The elongate tube can be positioned around the balloon catheter. The balloon catheter can be positioned through the opening of the elongate tube such that the elongate tube is around the balloon catheter. A distal portion and a proximal portion of the elongate tube can be coupled, for example bonded, to respective portions of the balloon catheter to provide an inflatable balloon circumferentially positioned around the balloon catheter. In some embodiments, the distal portion and the proximal portion of the elongate tube can be bonded to respective parts of the distal portion of the balloon catheter to provide an inflatable balloon circumferentially around the balloon catheter distal portion. In some embodiments, the first and second compositions can be extruded to form an elongate tube which can be cut into individual tubes having a desired length such that the individual tubes can be used to form respective inflatable balloons associated with corresponding balloon catheters.

Although the inflatable balloons are primarily described herein as being used for deployment into a pulmonary blood vessel, it will be understood that the inflatable balloons can be used for other purposes. The inflatable balloons can be deployed into other body chambers, lumens and/or vessels, including to facilitate collection of any number of parameters.

In some embodiments, an inflatable balloon can comprise a balloon wall which has undergone a pre-inflation process. For example, an inflatable balloon can be made using a balloon wall which was part of previously inflated balloon. At least a part of the previously inflated balloon can be used to form an inflatable balloon associated with a balloon catheter. A balloon wall which had undergone a pre-inflation process can demonstrate less plastic deformation when subsequently inflated, such as compared to a balloon wall which has never been previously inflated. Reduced plastic deformation of the balloon wall in subsequent inflations can result in less slack and/or bagginess when the inflatable balloon deflated after an inflation. Less slack and/or bagginess in the deflated state can facilitate retraction of the deflated balloon and the balloon catheter back into an introducer catheter.

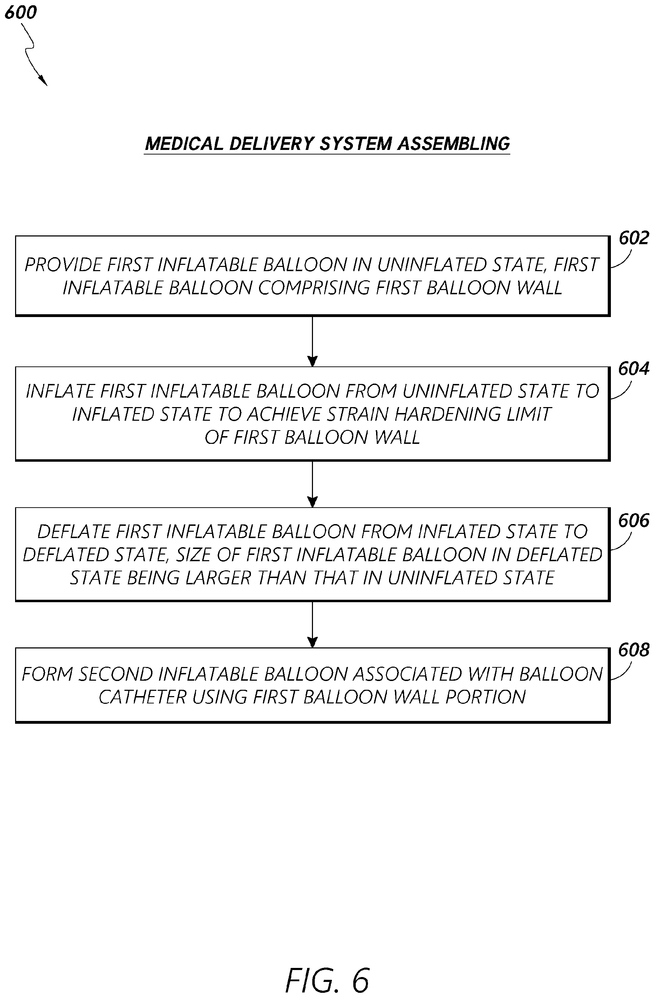

Figure 6 is a process flow diagram of an example of a process 600 for assembling a medical delivery system which includes an inflatable balloon comprising a balloon wall which has undergone a pre-inflation process. In block 602, the process 600 can involve providing a first inflatable balloon in an uninflated state, the first inflatable balloon comprising a first balloon wall. In some embodiments, the first balloon wall can be one layer. For example, the first balloon wall can be a single balloon wall layer balloon. In some embodiments, the first balloon wall can comprise a styrenic block co-polymer. In some embodiments, the first balloon wall can comprise styrene-isoprene-styrene (SIS), styrene-butadiene-styrene (SBS) layer or styrene-ethylene-butylene-styrene (SEBS). For example, the first balloon wall can be a styrene-isoprene-styrene (SIS) layer, a styrene-butadiene-styrene (SBS) layer or a styrene-ethylene-butylene-styrene (SEBS) layer. In some embodiments, the first inflatable balloon can be a single balloon wall layer balloon where the single balloon wall layer is a styrene-isoprene-styrene (SIS) layer, a styrene-butadiene-styrene (SBS) layer or a styrene-ethylene-butylene-styrene (SEBS) layer. In some embodiments, the first balloon wall can comprise polyurethane. For example, the first inflatable balloon can be a single balloon wall layer balloon where the single balloon wall layer comprises polyurethane.

In some embodiments, the first balloon wall can comprise more than one layer. In some embodiments, the first balloon wall can comprise two balloon wall layers. For example, the first balloon wall can have one or more configurations as described herein.

In block 604, the process 600 can involve inflating the first inflatable balloon from the uninflated state to an inflated state to achieve a strain hardening limit of the first balloon wall. The inflated state can comprise an inflated volume and the first balloon wall can at least partially define the inflated volume. For example, the first balloon wall can be the balloon wall for the first inflatable balloon, such that the first balloon wall defines the inflated volume within the first inflatable balloon.

In block 606, the process 600 can involve deflating the first inflatable balloon from the inflated state to a deflated state. The first inflatable balloon wall can demonstrate plastic deformation as a result of the inflation. A size of the first inflatable balloon in the deflated state can be larger than that in the uninflated state.

In some embodiments, the first inflatable balloon can comprise an elongate configuration. In some embodiments, the first inflatable balloon can comprise a cylindrical shape. For example, inflating the first balloon can comprise extending the first balloon along a longitudinal axis. The first balloon can inflate to a diameter based at least in part on the strain hardening property of the first balloon wall. In some embodiments, the first inflatable balloon can comprise a first elongate inflatable balloon. Inflating the first elongate balloon can be performed to achieve a strain hardening limit of the first balloon wall such that the first elongate balloon exhibits plastic deformation. In some embodiments, the first balloon can be irreversibly stretched along a lateral dimension, such as a diameter. The lateral dimension can be perpendicular or substantially perpendicular to the longitudinal axis of the first elongate balloon. The first elongate inflatable balloon in the deflated state can be larger than the first elongate inflatable balloon prior to the inflation. In some embodiments, the first elongate inflatable balloon in the deflated state can have a larger diameter than that in the uninflated state, including being about 10% to about 20% larger, including about 10% to about 15%, and about 15% to about 20% larger. For example, the first inflatable balloon in the deflated state can have a plastic deformation of about 10% to about 20%, including about 10% to about 15% and about 15% to about 20%.

The uninflated first elongate inflatable balloon can have an initial inner diameter. The deflated first elongate inflatable balloon can have a size larger than that prior to the inflation. For example, an inner diameter of the deflated first elongate inflatable balloon, such as a deflated inner diameter, can be larger than the initial inner diameter.

In some embodiments, the first inflatable balloon can have an initial inner diameter of about 0.050 inches (in) to about 0.060 inches (in), including about 0.055 inches (in). In some embodiments, the first inflatable balloon can have a deflated inner diameter of about 0.060 inches (in) to about 0.070 inches (in), including about 0.065 inches (in). In some embodiments, the initial inner diameter can be selected based on a target after deflation inner diameter. In some embodiments, the after inflation inner diameter can be about 0.2 inches (in) to about 0.3 inches (in). In some embodiments, the after inflation inner diameter can be about 0.045 inches (in) to about 0.085 inches (in), including about 0.060 inches (in) to about 0.085 inches (in).

In some embodiments, the balloon wall can have a total thickness of about 0.005 inches (in) to about 0.015 inches (in), including about 0.010 inches (in) to about 0.015 inches (in). In some embodiments, the balloon wall can have a total thickness of about 0.010 inches (in) to about 0.012 inches (in). In some embodiments, the balloon wall can have a total thickness of about 0.008 inches (in) to about 0.010 inches (in). In some embodiments, the balloon wall can have a total thickness of about 0.020 inches (in) to about 0.030 inches (in). In some embodiments, the wall thickness of the balloon wall in the deflated state can be similar to or the same as the initial wall thickness.

In some embodiments, the inflation can be performed under ambient conditions. For example, the inflation can be performed at around ambient temperature (e.g., about 20°C to about 25°C) and standard pressure (i.e., 1 atmosphere (atm)). In some embodiments, the inflation can be performed at an elevated temperature (e.g., about 25°C to about 40°C) and standard pressure (i.e., 1 atmosphere (atm)). In some embodiments, the inflation can be performed at around body temperature (e.g., about 37°C).

In some embodiments, the inflation can comprise pressurizing the first inflatable balloon with air and/or inert gas. In some embodiments, the inflation can be performed in a medium comprising water and/or isopropanol. For example, the medium can comprise about 30 wt% water and about 70 wt% isopropanol. In some embodiments, the medium can be about 30 wt% water and about 70 wt% isopropanol. The medium can be provided at a temperature of about 20°C to about 40°C. In some embodiments, the first inflatable balloon can be inflated while maintained within the medium. The first inflatable balloon can remain inflated for up to about 30 minutes after inflation prior to deflation. For example, the first inflatable balloon can remain in the inflated state within the medium for up to about 30 minutes after the inflation. The first inflatable balloon can be inflated while submerged in the medium and subsequently remain submerged within the medium for up to about 30 minutes after inflation. In some embodiments, the first inflatable balloon can be submerged in the medium for about 10 to about 30 minutes, including about 10 to about 20 minutes.

In block 608, the process 600 can involve forming a second inflatable balloon associated with a balloon catheter using a first balloon wall portion. After the first inflatable balloon is deflated, a portion of the first balloon wall can be used to form the second inflatable balloon. In some embodiments, the second inflatable balloon can be formed on a distal portion of the balloon catheter. Forming the second inflatable balloon can comprise positioning the first balloon wall portion around a predetermined part of the balloon catheter distal portion. A proximal portion and a distal portion of the first balloon wall portion can be coupled to the distal portion of the balloon catheter. A medial portion between the proximal portion and the distal portion can be expandable upon inflation. For example, the medial portion can be expanded when the second inflatable balloon is inflated such that the inflated volume of the second inflatable balloon can be defined at least in part by the medial portion. The inflated volume can be defined by an exterior surface portion of the balloon catheter distal portion and the balloon wall medial portion.

As described herein, in some embodiments, the first inflatable balloon can comprise a first elongate inflatable balloon. Forming the second inflatable balloon can comprise cutting the first elongate inflatable balloon to a desired length so as to provide the first balloon wall portion which can be used to form the second inflatable balloon. In some embodiments, end portions of the first balloon wall can be removed to provide the portion of the first balloon wall used to form the second inflatable balloon. In some embodiments, the first balloon wall can be cut along two paths parallel or substantially parallel to the lateral dimension and spaced apart from one another to provide the first balloon wall portion. For example, the first balloon wall portion can comprise a circumferential portion of the first elongate inflatable balloon and can have a longitudinal dimension shorter than that of the first elongate balloon. The first balloon wall portion can be positioned around the predetermined part of the balloon catheter distal portion. A proximal circumferential portion and a distal circumferential portion of the first balloon wall portion can be coupled to the distal portion of the balloon catheter. Any number of techniques can be used to couple the first balloon wall portion to the balloon catheter, including for example bonding. A medial circumferential portion between the proximal circumferential portion and the distal circumferential portion can be expandable upon inflation.

In some embodiments, the first elongate inflatable balloon can comprise a cylindrical shape. Cutting the first elongate inflatable balloon can comprise providing a cylindrically shaped balloon wall portion having a longitudinal dimension, such as a length, shorter than that of the first elongate inflatable balloon. For example, the portion of the first balloon wall used for the second inflatable balloon can have a tubular shape. The balloon catheter can be positioned within the lumen of the tubular first balloon wall portion. Proximal and distal circumferential portions of the tubular first balloon wall portion can be bonded to the balloon catheter, such as with an adhesive.

In some embodiments, the portion of the first balloon used to form the second inflatable balloon can have a length of about 0.2 inches (in) to about 0.5 inches (in), including about 0.2 inches (in) to about 0.4 inches (in). In some embodiments, the portion of the first balloon can have a length of about 0.3 inches (in).

In some embodiments, a process for assembling the medical delivery system can comprise forming the first balloon wall. Forming the balloon wall can comprise providing a first composition comprising styrene and one of isoprene or butadiene and extruding the first composition to provide the first balloon wall of the first inflatable balloon. The first balloon wall can comprise a styrenic block copolymer. In some embodiments, the first balloon wall can be a styrenic block copolymer layer as described herein. For example, providing the first composition can comprise providing a composition comprising about 5 mol % to about 15 mol % of styrene monomers. In some embodiments, the first composition can comprise polyurethane. In some embodiments, the first balloon wall can be a polyurethane layer as described herein.

In some embodiments, the first composition can comprise a surface tackiness reducing agent, the surface tackiness reducing agent comprising at least one of polytetrafluoroethylene (PTFE) and a radiopaque filler material. In some embodiments, the radiopaque filler material can comprise at least one of bismuth oxychloride (BiOCl), barium sulfate (BaSO₄), and bismuth subcarbonate ((BiO)₂CO₃). For example, the first composition can comprise a styrenic block copolymer and one or more surface tackiness reducing agents.

In some embodiments, the first balloon wall can comprise more than one balloon wall layer. For example, assembling the medical delivery system can comprise providing a first composition comprising styrene and one of isoprene or butadiene, and providing a second composition comprising styrene and one of isoprene or butadiene, the second composition being different from the first composition. The first composition and the second composition can be co-extruded to provide a first balloon wall comprising a first balloon wall layer and a second balloon wall layer, respectively. In some embodiments, the first balloon wall layer can be an inner layer of the first balloon wall. The second balloon wall layer can be an outer layer of the first balloon wall. The first balloon wall layer can have a Shore A hardness smaller than that of the second balloon wall layer. In some embodiments, the first and second balloon wall layers can comprise one or more compositions as described herein (e.g., as the first and second balloon wall layers described with reference to Figure 2).

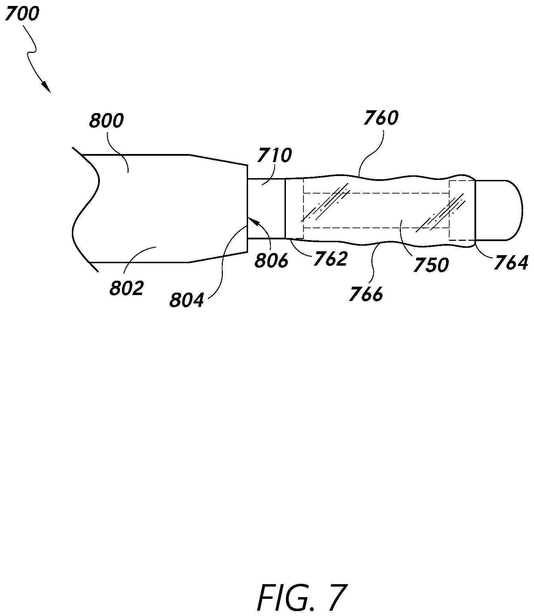

Figure 7 shows an example of a medical delivery system 700 comprising at least a portion assembled using the assembly process 600 described with reference to Figure 6. The medical delivery system 700 can comprise a balloon catheter 710, an inflatable balloon 750 associated with the balloon catheter 710 and an introducer catheter 800 configured to slidably receive at least a portion of the balloon catheter 710. The inflatable balloon 750 can be manufactured according to a process comprising one or more steps described with reference to Figure 6. For example, the inflatable balloon 750 can comprise a balloon wall 760 which has undergone a pre-inflation process. The inflatable balloon 750 can be within a distal portion 802 of the introducer catheter 800 while being delivered to a target site within the body. After positioning at the target site, the balloon catheter 710 can be translated distally relative to the introducer catheter 800 such that the inflatable balloon 750 can be released through the distal end 804 of the introducer catheter 800, including through a distal opening 806 at the distal end 804. The inflatable balloon 750 can then be inflated. As described herein, the pre-inflation process can be performed to plastically deform the balloon wall 760 such that the inflatable balloon 750 can exhibit reduced slack and/or bagginess after inflation and deflation. The reduced slack and/or bagginess can facilitate retraction of the balloon catheter 710 back into the introducer catheter 800. In some embodiments, the inflatable balloon 750 can demonstrate less slack and/or bagginess after an inflation and deflation at the target site as compared to an inflatable balloon which has never previously been inflated. Figure 7 shows the inflatable balloon 750 distal of the distal opening 806 and in a deflated state.

In some embodiments, the introducer catheter 800 can have an outer diameter less than about 0.5 French larger than that of the balloon catheter 710 with which the inflatable balloon 750 is associated. In some embodiments, the introducer catheter 800 can have the same or a similar outer diameter as that of the balloon catheter 710. For example, the balloon catheter 710 and the introducer catheter 800 can have the same French size. In some embodiments, the inflatable balloon 750 in a deflated state can be retracted back into the introducer catheter 800 having the same French size as the balloon catheter 710, such as without being damaged and/or getting stuck to the introducer catheter 800.

The medical delivery system 700 can comprise one or more other characteristics of the medical delivery system 100 described herein. For example, the inflatable balloon 750 can be associated with a distal portion 712 of the balloon catheter 710. For example, the inflatable balloon 750 can be circumferentially around the distal portion 712 of the balloon catheter 710. The balloon wall 760 can comprise a proximal portion 762 and a distal portion 764 coupled circumferentially to, such as bonded circumferentially to, respective parts of the balloon catheter distal portion 712. A medial portion 766 of the balloon wall 760 between the proximal and distal portions 762, 764 and an exterior surface 720 of the balloon catheter 710 can define the inflated volume.

### Additional Embodiments

Conditional language used herein, such as, among others, "can," "could," "might," "may," "e.g.," and the like, unless specifically stated otherwise, or otherwise understood within the context as used, is intended in its ordinary sense and is generally intended to convey that certain embodiments include, while other embodiments do not include, certain features, elements and/or steps. Thus, such conditional language is not generally intended to imply that features, elements and/or steps are in any way required for one or more embodiments or that one or more embodiments necessarily include logic for deciding, with or without author input or prompting, whether these features, elements and/or steps are included or are to be performed in any particular embodiment. The terms "comprising," "including," "having," and the like are synonymous, are used in their ordinary sense, and are used inclusively, in an open-ended fashion, and do not exclude additional elements, features, acts, operations, and so forth. Also, the term "or" is used in its inclusive sense (and not in its exclusive sense) so that when used, for example, to connect a list of elements, the term "or" means one, some, or all of the elements in the list. Conjunctive language such as the phrase "at least one of X, Y and Z," unless specifically stated otherwise, is understood with the context as used in general to convey that an item, term, element, etc. may be either X, Y or Z. Thus, such conjunctive language is not generally intended to imply that certain embodiments require at least one of X, at least one of Y and at least one of Z to each be present.

It should be appreciated that in the above description of embodiments, various features are sometimes grouped together in a single embodiment, Figure, or description thereof for the purpose of streamlining the disclosure and aiding in the understanding of one or more of the various inventive aspects. This method of disclosure, however, is not to be interpreted as reflecting an intention that any claim require more features than are expressly recited in that claim.

It should be understood that certain ordinal terms (e.g., "first" or "second") may be provided for ease of reference and do not necessarily imply physical characteristics or ordering. Therefore, as used herein, an ordinal term (e.g., "first," "second," "third," etc.) used to modify an element, such as a structure, a component, an operation, etc., does not necessarily indicate priority or order of the element with respect to any other element, but rather may generally distinguish the element from another element having a similar or identical name (but for use of the ordinal term). In addition, as used herein, indefinite articles ("a" and "an") may indicate "one or more" rather than "one." Further, an operation performed "based on" a condition or event may also be performed based on one or more other conditions or events not explicitly recited.

Unless otherwise defined, all terms (including technical and scientific terms) used herein have the same meaning as commonly understood by one of ordinary skill in the art to which example embodiments belong. It be further understood that terms, such as those defined in commonly used dictionaries, should be interpreted as having a meaning that is consistent with their meaning in the context of the relevant art and not be interpreted in an idealized or overly formal sense unless expressly so defined herein.

The spatially relative terms "outer," "inner," "upper," "lower," "below," "above," "vertical," "horizontal," and similar terms, may be used herein for ease of description to describe the relations between one element or component and another element or component as illustrated in the drawings. It be understood that the spatially relative terms are intended to encompass different orientations of the device in use or operation, in addition to the orientation depicted in the drawings. For example, in the case where a device shown in the drawing is turned over, the device positioned "below" or "beneath" another device may be placed "above" another device. Accordingly, the illustrative term "below" may include both the lower and upper positions. The device may also be oriented in the other direction, and thus the spatially relative terms may be interpreted differently depending on the orientations.

Unless otherwise expressly stated, comparative and/or quantitative terms, such as "less," "more," "greater," and the like, are intended to encompass the concepts of equality. For example, "less" can mean not only "less" in the strictest mathematical sense, but also, "less than or equal to."

## Claims

1. A medical delivery system comprising:
a balloon catheter; and
an inflatable balloon associated with a portion of the balloon catheter, the inflatable balloon comprising a balloon wall at least partially defining an inflated volume of the inflatable balloon, the balloon wall comprising a first balloon wall layer and a second balloon wall layer, the first balloon wall layer comprising a Shore A durometer hardness value smaller than that of the second balloon wall layer, and the first balloon wall layer being an inner layer of the balloon wall relative to the second balloon wall layer;
wherein the first balloon wall layer is about 70% to about 85% of a total thickness of the balloon wall.

2. The system of claim 1, wherein the first balloon wall layer comprises a Shore A durometer hardness value of about 20 to about 45 and the second balloon wall layer comprises a Shore A durometer hardness value of about 25 to about 50.

3. The system of claim 1 or 2, wherein the balloon wall is the first balloon wall layer and the second balloon wall layer.

4. The system of any one of claims 1 to 3, wherein at least one of the first balloon wall layer and the second balloon wall layer comprises a styrenic block copolymer,
optionally wherein the styrenic block copolymer:
a) is styrene-isoprene-styrene (SIS) or is one of styrene-butadiene-styrene (SBS) or styrene-ethylene-butylene-styrene (SEBS); and/or
b) comprises about 5 mol % to about 15 mol % of styrene monomers.

5. The system of any one of claims 1 to 4, wherein the balloon wall comprises a surface tackiness reducing agent,
optionally wherein:
a) the surface tackiness reducing agent comprises polytetrafluoroethylene (PTFE), optionally wherein the balloon wall comprises about 5 wt% to about 10 wt % of the polytetrafluoroethylene (PTFE);
b) the surface tackiness reducing agent comprises a radiopaque filler material, optionally wherein the balloon wall comprises about 10 wt% to about 40 wt % of the radiopaque filler material, and optionally wherein the radiopaque filler material comprises at least one of bismuth oxychloride (BiOCl), barium sulfate (BaSO₄), and bismuth subcarbonate ((BiO)₂CO₃); and/or
c) the second balloon wall layer comprises the surface tackiness reducing agent.

6. The system of any one of claims 1 to 5, wherein at least one of the first balloon wall layer and the second balloon wall layer comprises polyurethane.

7. The system of any one of claims 1 to 6, wherein the inflatable balloon is around a distal portion of the balloon catheter,
optionally wherein the balloon wall;
a) comprises a proximal portion and a distal portion, both the proximal portion and the distal portion being bonded circumferentially around the distal portion of the balloon catheter to provide a circumferential balloon around the distal portion; and/or
b) is bonded circumferentially around the distal portion of the balloon catheter, the inflated volume being defined by a circumferential wall portion of the distal portion to which the balloon wall is bonded and the balloon wall.

8. The system of any one of claims 1 to 7, wherein the balloon wall comprises a third balloon wall layer, the third balloon wall layer being an inner balloon wall layer, the second balloon wall layer being an outer balloon wall layer, and the first balloon wall layer being an intermediate balloon wall layer between the third balloon wall layer and the second balloon wall layer,
optionally wherein the first balloon wall layer and the second balloon wall layer each comprise a respective styrenic block copolymer, and wherein the third balloon wall layer comprises polyurethane.

9. The system of any one of claims 1 to 8 further comprising an introducer catheter, at least the portion of the balloon catheter being configured to be slidably received within a balloon catheter lumen of the introducer catheter,
optionally wherein the introducer catheter has an outer diameter less than about 0.5 French larger than that of the balloon catheter, and
optionally wherein the portion of the balloon catheter associated with the inflatable balloon and the inflatable balloon in a deflated state are configured to be extendable and retractable through an opening at a distal end of the introducer catheter.

10. A method of assembling a medical balloon catheter system, the method comprising:
providing a first inflatable balloon in an uninflated state, the first inflatable balloon comprising a first balloon wall;
inflating the first inflatable balloon from the uninflated state to an inflated state to achieve a strain hardening limit of the first balloon wall, the inflated state comprising an inflated volume and the first balloon wall at least partially defining the inflated volume;
deflating the first inflatable balloon from the inflated state to a deflated state, a size of the first inflatable balloon in the deflated state being larger than that in the uninflated state; and
forming a second inflatable balloon associated with a balloon catheter using a first balloon wall portion;
wherein the first balloon wall comprises a first balloon wall layer and a second balloon wall layer, the first balloon wall layer comprising a Shore A durometer hardness value smaller than that of the second balloon wall layer, and the first balloon wall layer being an inner layer of the balloon wall relative to the second balloon wall layer; and
wherein the first balloon wall layer is about 70% to about 85% of a total thickness of the balloon wall.

11. The method of claim 10, wherein forming the second inflatable balloon comprises positioning the first balloon wall portion around a distal portion of the balloon catheter, and coupling a proximal circumferential portion and a distal circumferential portion of the first balloon wall portion to the distal portion of the balloon catheter, a medial circumferential portion of the first balloon wall portion being expandable upon inflation.

12. The method of claim 10 or 11, wherein providing the first inflatable balloon comprises providing a first elongate inflatable balloon, the first elongate inflatable balloon comprising a longitudinal dimension and a lateral dimension perpendicular to the longitudinal dimension, and wherein forming the second inflatable balloon comprises:
cutting the first elongate inflatable balloon along two paths parallel to the lateral dimension and spaced apart from one another to provide the first balloon wall portion, the first balloon wall portion comprising a circumferential portion of the first elongate inflatable balloon and having a longitudinal dimension shorter than that of the first elongate balloon;
positioning the first balloon wall portion around a distal portion of the balloon catheter; and
bonding a proximal circumferential portion and a distal circumferential portion of the first balloon wall portion to the distal portion of the balloon catheter, a medial circumferential portion of the first balloon wall portion being expandable upon inflation,
optionally wherein providing the first elongate inflatable balloon comprises providing a cylindrical balloon, and wherein cutting the first elongate inflatable balloon comprises providing a cylindrical balloon wall portion having a longitudinal dimension shorter than that of the first elongate inflatable balloon.

13. The method of any one of claims 10 to 12, further comprising inserting a distal portion of the balloon catheter through a balloon catheter lumen of an introducer catheter, the introducer catheter comprising an outer diameter size less than about 0.5 French larger than that of the balloon catheter.

14. The method of any one of claims 10 to 13, wherein providing the first inflatable balloon comprises providing a first elongate inflatable balloon, the first elongate inflatable balloon comprising a longitudinal dimension and a lateral dimension perpendicular to the longitudinal dimension, and wherein deflating the first inflatable balloon from the inflated state to the deflated state comprises deflating the first elongate inflatable balloon from the inflated state to a deflated state comprising a lateral dimension about 15% to about 20% larger than that of a lateral dimension of the first elongate inflatable balloon in the uninflated state.

15. The method of any one of claims 9 to 14, further comprising:
providing a first composition comprising styrene and one of isoprene or butadiene;
providing a second composition comprising styrene and one of isoprene or butadiene, the second composition being different from the first composition; and
co-extruding the first composition and the second composition to provide the first balloon wall.

## Patentansprüche

1. Medizinisches Zuführsystem, umfassend:
einen Ballonkatheter; und
einen aufblasbaren Ballon, der mit einem Abschnitt des Ballonkatheters assoziiert ist, wobei der aufblasbare Ballon eine Ballonwand umfasst, die zumindest teilweise ein aufgeblasenes Volumen des aufblasbaren Ballons definiert, wobei die Ballonwand eine erste Ballonwandschicht und eine zweite Ballonwandschicht umfasst, wobei die erste Ballonwandschicht einen Shore-A-Durometerhärtewert umfasst, der kleiner als der der zweiten Ballonwandschicht ist, und wobei die erste Ballonwandschicht relativ zur zweiten Ballonwandschicht eine Innenschicht der Ballonwand ist;
wobei die erste Ballonwandschicht etwa 70 % bis etwa 85 % einer Gesamtdicke der Ballonwand beträgt.

2. System nach Anspruch 1, wobei die erste Ballonwandschicht einen Shore-A-Durometerhärtewert von etwa 20 bis etwa 45 umfasst und die zweite Ballonwandschicht einen Shore-A-Durometerhärtewert von etwa 25 bis etwa 50 umfasst.

3. System nach Anspruch 1 oder 2, wobei die Ballonwand die erste Ballonwandschicht und die zweite Ballonwandschicht ist.

4. System nach einem der Ansprüche 1 bis 3, wobei mindestens eine der ersten Ballonwandschicht und der zweiten Ballonwandschicht ein Styrolblockcopolymer umfasst,
optional wobei das Styrolblockcopolymer:
a) Styrol-Isopren-Styrol (SIS) ist oder eines von Styrol-Butadien-Styrol (SBS) oder Styrol-Ethylen-Butylen-Styrol (SEBS) ist; und/oder
b) etwa 5 Mol-% bis etwa 15 Mol-% Styrolmonomere umfasst.

5. System nach einem der Ansprüche 1 bis 4, wobei die Ballonwand ein Oberflächenklebrigkeitsreduzierungsmittel umfasst,
optional wobei:
a) das Oberflächenklebrigkeitsreduzierungsmittel Polytetrafluorethylen (PTFE) umfasst, optional wobei die Ballonwand etwa 5 Gew.-% bis etwa 10 Gew.-% des Polytetrafluorethylens (PTFE) umfasst;
b) das Oberflächenklebrigkeitsreduzierungsmittel ein röntgendichtes Füllmaterial umfasst, optional wobei die Ballonwand etwa 10 Gew.-% bis etwa 40 Gew.-% des röntgendichten Füllmaterials umfasst, und optional wobei das röntgendichte Füllmaterial mindestens eines von Bismutoxychlorid (BiOCl), Bariumsulfat (BaSO₄) und Bismutsubcarbonat ((BiO)₂CO₃) umfasst; und/oder
c) die zweite Ballonwandschicht das Oberflächenklebrigkeitsreduzierungsmittel umfasst.

6. System nach einem der Ansprüche 1 bis 5, wobei mindestens eine der ersten Ballonwandschicht und der zweiten Ballonwandschicht Polyurethan umfasst.

7. System nach einem der Ansprüche 1 bis 6, wobei sich der aufblasbare Ballon um einen distalen Abschnitt des Ballonkatheters herum befindet,
optional wobei die Ballonwand
a) einen proximalen Abschnitt und einen distalen Abschnitt umfasst, wobei sowohl der proximale Abschnitt als auch der distale Abschnitt umlaufend um den distalen Abschnitt des Ballonkatheters gebondet sind, um einen um den distalen Abschnitt umlaufenden Ballon bereitzustellen; und/oder
b) umlaufend um den distalen Abschnitt des Ballonkatheters gebondet ist, wobei das aufgeblasene Volumen durch einen Umfangswandabschnitt des distalen Abschnitts, an den die Ballonwand gebondet ist, und die Ballonwand definiert ist.

8. System nach einem der Ansprüche 1 bis 7, wobei die Ballonwand eine dritte Ballonwandschicht umfasst, wobei die dritte Ballonwandschicht eine innere Ballonwandschicht ist, die zweite Ballonwandschicht eine äußere Ballonwandschicht ist und die erste Ballonwandschicht eine Zwischenballonwandschicht zwischen der dritten Ballonwandschicht und der zweiten Ballonwandschicht ist,
optional wobei die erste Ballonwandschicht und die zweite Ballonwandschicht jeweils ein jeweiliges Styrolblockcopolymer umfassen, und wobei die dritte Ballonwandschicht Polyurethan umfasst.

9. System nach einem der Ansprüche 1 bis 8, ferner umfassend einen Einführkatheter, wobei zumindest der Abschnitt des Ballonkatheters dazu ausgelegt ist, verschiebbar in einem Ballonkatheterlumen des Einführkatheters aufgenommen zu werden,
optional wobei der Einführkatheter einen Außendurchmesser aufweist, der weniger als etwa 0,5 Charrière größer als der des Ballonkatheters ist, und
optional wobei der Abschnitt des Ballonkatheters, der mit dem aufblasbaren Ballon assoziiert ist, und der aufblasbare Ballon in einem entleerten Zustand dazu ausgelegt sind, durch eine Öffnung an einem distalen Ende des Einführkatheters ausziehbar und einziehbar zu sein.

10. Verfahren zum Zusammenbauen eines medizinischen Ballonkathetersystems, wobei das Verfahren Folgendes umfasst:
Bereitstellen eines ersten aufblasbaren Ballons in einem nicht aufgeblasenen Zustand, wobei der erste aufblasbare Ballon eine erste Ballonwand umfasst;
Aufblasen des ersten aufblasbaren Ballons von dem nicht aufgeblasenen Zustand in einen aufgeblasenen Zustand, um eine Kaltverfestigungsgrenze der ersten Ballonwand zu erreichen, wobei der aufgeblasene Zustand ein aufgeblasenes Volumen umfasst und die erste Ballonwand zumindest teilweise das aufgeblasene Volumen definiert;
Entleeren des ersten aufblasbaren Ballons von dem aufgeblasenen Zustand in einen entleerten Zustand, wobei eine Größe des ersten aufblasbaren Ballons im entleerten Zustand größer als die im nicht aufgeblasenen Zustand ist; und
Bilden eines zweiten aufblasbaren Ballons, der mit einem Ballonkatheter assoziiert ist, unter Verwendung eines ersten Ballonwandabschnitts;
wobei die erste Ballonwand eine erste Ballonwandschicht und eine zweite Ballonwandschicht umfasst, wobei die erste Ballonwandschicht einen Shore-A-Durometerhärtewert umfasst, der kleiner als der der zweiten Ballonwandschicht ist, und wobei die erste Ballonwandschicht relativ zur zweiten Ballonwandschicht eine Innenschicht der Ballonwand ist; und
wobei die erste Ballonwandschicht etwa 70 % bis etwa 85 % einer Gesamtdicke der Ballonwand beträgt.

11. Verfahren nach Anspruch 10, wobei Bilden des zweiten aufblasbaren Ballons Positionieren des ersten Ballonwandabschnitts um einen distalen Abschnitt des Ballonkatheters herum und Koppeln eines proximalen Umfangsabschnitts und eines distalen Umfangsabschnitts des ersten Ballonwandabschnitts mit dem distalen Abschnitt des Ballonkatheters umfasst, wobei ein medialer Umfangsabschnitt des ersten Ballonwandabschnitts beim Aufblasen expandierbar ist.

12. Verfahren nach Anspruch 10 oder 11, wobei Bereitstellen des ersten aufblasbaren Ballons Bereitstellen eines ersten länglichen aufblasbaren Ballons umfasst, wobei der erste längliche aufblasbare Ballon eine Längsabmessung und eine Querabmessung senkrecht zur Längsabmessung umfasst, und wobei Bilden des zweiten aufblasbaren Ballons Folgendes umfasst:
Schneiden des ersten länglichen aufblasbaren Ballons entlang zweier Pfade, die parallel zur Querabmessung verlaufen und voneinander beabstandet sind, um den ersten Ballonwandabschnitt bereitzustellen, wobei der erste Ballonwandabschnitt einen Umfangsabschnitt des ersten länglichen aufblasbaren Ballons umfasst und eine Längsabmessung aufweist, die kürzer als die des ersten länglichen Ballons ist;
Positionieren des ersten Ballonwandabschnitts um einen distalen Abschnitt des Ballonkatheters herum; und
Bonden eines proximalen Umfangsabschnitts und eines distalen Umfangsabschnitts des ersten Ballonwandabschnitts an den distalen Abschnitt des Ballonkatheters, wobei ein medialer Umfangsabschnitt des ersten Ballonwandabschnitts beim Aufblasen expandierbar ist,
optional wobei Bereitstellen des ersten länglichen aufblasbaren Ballons Bereitstellen eines zylindrischen Ballons umfasst, und wobei Schneiden des ersten länglichen aufblasbaren Ballons Bereitstellen eines zylindrischen Ballonwandabschnitts umfasst, der eine Längsabmessung aufweist, die kürzer als die des ersten länglichen aufblasbaren Ballons ist.

13. Verfahren nach einem der Ansprüche 10 bis 12, ferner umfassend Einführen eines distalen Abschnitts des Ballonkatheters durch ein Ballonkatheterlumen eines Einführkatheters, wobei der Einführkatheter eine Außendurchmessergröße umfasst, die weniger als etwa 0,5 Charrière größer als die des Ballonkatheters ist.

14. Verfahren nach einem der Ansprüche 10 bis 13, wobei Bereitstellen des ersten aufblasbaren Ballons Bereitstellen eines ersten länglichen aufblasbaren Ballons umfasst, wobei der erste längliche aufblasbare Ballon eine Längsabmessung und eine Querabmessung senkrecht zur Längsabmessung umfasst, und wobei Entleeren des ersten aufblasbaren Ballons von dem aufgeblasenen Zustand in den entleerten Zustand Entleeren des ersten länglichen aufblasbaren Ballons von dem aufgeblasenen Zustand in einen entleerten Zustand umfasst, der eine Querabmessung umfasst, die etwa 15 % bis etwa 20 % größer als die einer Querabmessung des ersten länglichen aufblasbaren Ballons im nicht aufgeblasenen Zustand ist.

15. Verfahren nach einem der Ansprüche 9 bis 14, ferner umfassend:
Bereitstellen einer ersten Zusammensetzung, die Styrol und eines von Isopren oder Butadien umfasst;
Bereitstellen einer zweiten Zusammensetzung, die Styrol und eines von Isopren oder Butadien umfasst, wobei sich die zweite Zusammensetzung von der ersten Zusammensetzung unterscheidet; und
Koextrudieren der ersten Zusammensetzung und der zweiten Zusammensetzung, um die erste Ballonwand bereitzustellen.

## Revendications

1. Système médical de délivrance comprenant :
un cathéter à ballonnet ; et
un ballonnet gonflable associé à une partie du cathéter à ballonnet, le ballonnet gonflable comprenant une paroi de ballonnet définissant au moins partiellement un volume gonflé du ballonnet gonflable, la paroi de ballonnet comprenant une première couche de paroi de ballonnet et une deuxième couche de paroi de ballonnet, la première couche de paroi de ballonnet présentant une valeur de dureté au duromètre Shore A inférieure à celle de la deuxième couche de paroi de ballonnet, et la première couche de paroi de ballonnet étant une couche interne de la paroi de ballonnet par rapport à la deuxième couche de paroi de ballonnet ;
dans lequel la première couche de paroi de ballonnet constitue environ 70 % à environ 85 % d'une épaisseur totale de la paroi de ballonnet.

2. Système selon la revendication 1, dans lequel la première couche de paroi de ballonnet présente une valeur de dureté au duromètre Shore A d'environ 20 à environ 45 et la deuxième couche de paroi de ballonnet présente une valeur de dureté au duromètre Shore A d'environ 25 à environ 50.

3. Système selon la revendication 1 ou 2, dans lequel la paroi de ballonnet est la première couche de paroi de ballonnet et la deuxième couche de paroi de ballonnet.

4. Système selon l'une quelconque des revendications 1 à 3, dans lequel au moins une couche parmi la première couche de paroi de ballonnet et la deuxième couche de paroi de ballonnet comprend un copolymère à blocs styréniques,
éventuellement dans lequel le copolymère à blocs styréniques :
a) est le styrène-isoprène-styrène (SIS) ou est soit le styrène-butadiène-styrène (SBS), soit le styrène-éthylène-butylène-styrène (SEBS) ; et/ou
b) comprend environ 5 % en moles à environ 15 % en moles de monomères de styrène.

5. Système selon l'une quelconque des revendications 1 à 4, dans lequel la paroi de ballonnet comprend un agent réduisant l'adhésivité de surface,
éventuellement dans lequel :
a) l'agent réduisant l'adhésivité de surface comprend du polytétrafluoroéthylène (PTFE), éventuellement dans lequel la paroi de ballonnet comprend environ 5 % en poids à environ 10 % en poids du polytétrafluoroéthylène (PTFE) ;
b) l'agent réduisant l'adhésivité de surface comprend un matériau de charge radio-opaque, éventuellement dans lequel la paroi de ballonnet comprend environ 10 % en poids à environ 40 % en poids du matériau de charge radio-opaque, et éventuellement dans lequel le matériau de charge radio-opaque comprend au moins un composé parmi l'oxychlorure de bismuth (BiOCl), le sulfate de baryum (BaSO₄), et le sous-carbonate de bismuth ((BiO)₂CO₃) ; et/ou
c) la deuxième couche de paroi de ballonnet comprend l'agent réduisant l'adhésivité de surface.

6. Système selon l'une quelconque des revendications 1 à 5, dans lequel au moins une couche parmi la première couche de paroi de ballonnet et la deuxième couche de paroi de ballonnet comprend du polyuréthane.

7. Système selon l'une quelconque des revendications 1 à 6, dans lequel le ballonnet gonflable se situe autour d'une partie distale du cathéter à ballonnet, éventuellement dans lequel la paroi de ballonnet ;
a) comprend une partie proximale et une partie distale, la partie proximale et la partie distale étant toutes deux liées circonférentiellement autour de la partie distale du cathéter à ballonnet pour constituer un ballonnet circonférentiel autour de la partie distale ; et/ou
b) est liée circonférentiellement autour de la partie distale du cathéter à ballonnet, le volume gonflé étant défini par une partie de paroi circonférentielle de la partie distale à laquelle la paroi de ballonnet est liée et la paroi de ballonnet.

8. Système selon l'une quelconque des revendications 1 à 7, dans lequel la paroi de ballonnet comprend une troisième couche de paroi de ballonnet, la troisième couche de paroi de ballonnet étant une couche de paroi de ballonnet interne, la deuxième couche de paroi de ballonnet étant une couche de paroi de ballonnet externe, et la première couche de paroi de ballonnet étant une couche de paroi de ballonnet intermédiaire entre la troisième couche de paroi de ballonnet et la deuxième couche de paroi de ballonnet,
éventuellement dans lequel la première couche de paroi de ballonnet et la deuxième couche de paroi de ballonnet comprennent chacune un copolymère à blocs styréniques respectif, et dans lequel la troisième couche de paroi de ballonnet comprend du polyuréthane.

9. Système selon l'une quelconque des revendications 1 à 8 comprenant en outre un cathéter d'introduction, au moins la partie du cathéter à ballonnet étant conçue pour être reçue par glissement à l'intérieur d'une lumière de cathéter à ballonnet du cathéter d'introduction,
éventuellement dans lequel le cathéter d'introduction a un diamètre extérieur supérieur de moins d'environ 0,5 French à celui du cathéter à ballonnet, et
éventuellement dans lequel la partie du cathéter à ballonnet associée au ballonnet gonflable et le ballonnet gonflable dans un état dégonflé sont conçus pour être extensibles et rétractables à travers une ouverture à une extrémité distale du cathéter d'introduction.

10. Procédé d'assemblage d'un système médical de cathéter à ballonnet, le procédé comprenant :
l'obtention d'un premier ballonnet gonflable dans un état non gonflé, le premier ballonnet gonflable comprenant une première paroi de ballonnet ;
le gonflage du premier ballonnet gonflable de l'état non gonflé à un état gonflé pour atteindre une limite d'écrouissage de la première paroi de ballonnet, l'état gonflé comprenant un volume gonflé et la première paroi de ballonnet définissant au moins partiellement le volume gonflé ;
le dégonflage du premier ballonnet gonflable de l'état gonflé à un état dégonflé, une taille du premier ballonnet gonflable dans l'état dégonflé étant plus grande que dans l'état non gonflé ; et
la formation d'un deuxième ballonnet gonflable associé à un cathéter à ballonnet au moyen d'une première partie de paroi de ballonnet ;
dans lequel la première paroi de ballonnet comprend une première couche de paroi de ballonnet et une deuxième couche de paroi de ballonnet, la première couche de paroi de ballonnet présentant une valeur de dureté au duromètre Shore A inférieure à celle de la deuxième couche de paroi de ballonnet, et la première couche de paroi de ballonnet étant une couche interne de la paroi de ballonnet par rapport à la deuxième couche de paroi de ballonnet ; et
dans lequel la première couche de paroi de ballonnet constitue environ 70 % à environ 85 % d'une épaisseur totale de la paroi de ballonnet.

11. Procédé selon la revendication 10, dans lequel la formation du deuxième ballonnet gonflable comprend le positionnement de la première partie de paroi de ballonnet autour d'une partie distale du cathéter à ballonnet, et le couplage d'une partie circonférentielle proximale et d'une partie circonférentielle distale de la première partie de paroi de ballonnet à la partie distale du cathéter à ballonnet, une partie circonférentielle médiane de la première partie de paroi de ballonnet étant extensible au gonflage.

12. Procédé selon la revendication 10 ou 11, dans lequel l'obtention du premier ballonnet gonflable comprend l'obtention d'un premier ballonnet gonflable allongé, le premier ballonnet gonflable allongé comprenant une dimension longitudinale et une dimension latérale perpendiculaire à la dimension longitudinale, et dans lequel la formation du deuxième ballonnet gonflable comprend :
la découpe du premier ballonnet gonflable allongé le long de deux trajectoires parallèles à la dimension latérale et espacées l'une de l'autre pour obtenir la première partie de paroi de ballonnet, la première partie de paroi de ballonnet comprenant une partie circonférentielle du premier ballonnet gonflable allongé et ayant une dimension longitudinale plus courte que celle du premier ballonnet allongé ;
le positionnement de la première partie de paroi de ballonnet autour d'une partie distale du cathéter à ballonnet ; et
la liaison d'une partie circonférentielle proximale et d'une partie circonférentielle distale de la première partie de paroi de ballonnet à la partie distale du cathéter à ballonnet, une partie circonférentielle médiane de la première partie de paroi de ballonnet étant extensible au gonflage,
éventuellement dans lequel l'obtention du premier ballonnet gonflable allongé comprend l'obtention d'un ballonnet cylindrique, et dans lequel la découpe du premier ballonnet gonflable allongé comprend l'obtention d'une partie de paroi de ballonnet cylindrique ayant une dimension longitudinale plus courte que celle du premier ballonnet gonflable allongé.

13. Procédé selon l'une quelconque des revendications 10 à 12, comprenant en outre l'insertion d'une partie distale du cathéter à ballonnet à travers une lumière de cathéter à ballonnet d'un cathéter d'introduction, le cathéter d'introduction présentant une taille en diamètre extérieur supérieure de moins d'environ 0,5 French à celle du cathéter à ballonnet.

14. Procédé selon l'une quelconque des revendications 10 à 13, dans lequel l'obtention du premier ballonnet gonflable comprend l'obtention d'un premier ballonnet gonflable allongé, le premier ballonnet gonflable allongé comprenant une dimension longitudinale et une dimension latérale perpendiculaire à la dimension longitudinale, et dans lequel le dégonflage du premier ballonnet gonflable de l'état gonflé à l'état dégonflé comprend le dégonflage du premier ballonnet gonflable allongé de l'état gonflé à un état dégonflé comprenant une dimension latérale supérieure d'environ 15 % à environ 20 % à celle d'une dimension latérale du premier ballonnet gonflable allongé dans l'état non gonflé.

15. Procédé selon l'une quelconque des revendications 9 à 14, comprenant en outre :
l'obtention d'une première composition comprenant du styrène, et soit de l'isoprène, soit du butadiène ;
l'obtention d'une deuxième composition comprenant du styrène, et soit de l'isoprène, soit du butadiène, la deuxième composition étant différente de la première composition ; et
la coextrusion de la première composition et de la deuxième composition pour obtenir la première paroi de ballonnet.
